# DEMANDE DE BREVET EUROPEEN

(11) **EP 4 717 189 A1**
(43) Date de publication de la demande: **01.04.2026**
(21) Numéro de dépôt: 25204115.7
(22) Date de dépôt: 23.09.2025
(51) Int. Cl.: A61B 10/00

(54) **CIRCUIT FLUIDIQUE AVEC SECTION DE CHANGEMENT DE SENS POUR ANALYSEUR D'URINE**

(30) Priorité: 26.09.2024 FR 2410308
(71) Demandeur: Withings, 92130 Issy-Les-Moulineaux (FR)
(72) Inventeur: Léger, Charlotte, 92130 Issy-les-Moulineaux (FR); Merlot, Antoine, 92130 Issy-les-Moulineaux (FR); Bendjoudi, Aniss, 92130 Issy-les-Moulineaux (FR)
(74) Mandataire: Withings IP

(57) **Abrégé**

La présente description se rapport à une station (200) d'analyse d'urine (100) comprenant un boitier, configuré pour être placé entièrement dans une cuvette de toilettes et pour recevoir un jet d'urine, le boitier étant configuré pour recevoir à l'intérieur au moins une région d'analyse apte à recevoir de l'urine pour analyse, un réservoir d'urine (524), configuré pour recevoir de l'urine issue d'une miction d'un utilisateur, un circuit fluidique (600, 900, 1000, 1100) à l'intérieur du boitier pour la circulation d'urine dans la station depuis le réservoir d'urine vers une région d'analyse, un analyseur (230), monté à l'intérieur du boitier (204), et configuré pour obtenir une information relative à l'urine dans la région d'analyse (508).

## Description

La présente invention concerne le dispositif d'analyse d'urine et notamment les dispositifs d'analyse d'urine comprenant un boitier configuré pour être placé entièrement dans une cuvette de toilettes.

### Etat de la technique

De nombreux paramètres biologiques sont identifiables dans l'urine d'un individu. Par exemple, des problèmes de santé tels qu'une infection des voies urinaires, un diabète ou une insuffisance rénale peuvent être détectés à partir d'un échantillon d'urine. L'échantillon d'urine peut également refléter la qualité d'un régime alimentaire, identifier une période de fertilité ou de grossesse et détecter la consommation de drogues ou de tabac. Il est donc intéressant de surveiller périodiquement divers paramètres biologiques.

Le document WO2021/175909 décrit un dispositif pour l'analyse d'urine. Le dispositif se loge dans les toilettes et recueille un échantillon d'urine avant d'effectuer une analyse optique. Le dispositif comprend une station et une cartouche, également appelée support rotatif, qui peut être retirée et remplacée de la station. La cartouche contient des bandelettes urinaires, c'est-à-dire des bandelettes enduites ou imprégnées d'un réactif réagissant avec l'urine.

Une difficulté liée aux dispositifs est la contamination de l'urine par différents composants et notamment de l'urine non liée à la miction précédent l'analyse (urine d'une autre personne ou urine d'une miction précédente, qui présente une composition différente et qui ne permet plus une analyse pertinente). On parlera de cross-contamination (anglicisme commun, traduisible par contamination croisée en français).

### Résumé de l'invention

La présente description propose donc une station d'analyse d'urine permettant de limiter les effets de cross-contamination.

Plusieurs solutions permettant de limiter les effets de cross-contamination vont être présentés. Ces solutions ne sont pas exclusives les unes des autres et peuvent être cumulées.

Selon un aspect, dit « injecteur-préleveur », la description se rapporte à une station d'analyse d'urine comprenant :
- un boitier configuré pour être placé entièrement dans une cuvette de toilettes et pour recevoir un jet d'urine, le boitier étant configuré pour recevoir à l'intérieur au moins une région d'analyse apte à recevoir de l'urine pour analyse,
- un réservoir d'urine, configuré pour recevoir de l'urine issue d'une miction d'un utilisateur,
- un circuit fluidique à l'intérieur du boitier pour la circulation d'urine dans la station depuis le réservoir d'urine vers une région d'analyse, dans lequel le circuit fluidique comprend une extrémité d'injection configurée pour injecter de l'urine dans une région d'analyse et une extrémité de prélèvement configuré pour prélever de l'urine depuis le réservoir et la faire pénétrer dans le circuit fluidique,
- un analyseur, monté à l'intérieur du boitier, et configuré pour obtenir une information relative à l'urine dans la région d'analyse,

Selon cet aspect « injecteur-préleveur », l'extrémité d'injection est utilisée comme extrémité de prélèvement. De la sorte l'extrémité d'injection est rincée par de l'urine de l'utilisateur avant l'injection.

Dans un mode de réalisation, l'extrémité d'injection est mobile entre notamment une position d'injection, durant laquelle l'extrémité d'injection injecte de l'urine dans la région d'analyse, et une position de prélèvement, durant laquelle l'extrémité d'injection prélève de l'urine dans le réservoir.

Dans un mode de réalisation, le réservoir et l'au moins une région d'analyse sont agencés à proximité dans la station, par exemple dans l'alignement d'un axe de translation de l'extrémité d'injection, qui est donc mobile.

Dans un mode de réalisation, l'extrémité d'injection est une seringue.

Dans un mode de réalisation, la station comprend une pompe configurée pour faire circuler l'urine dans le circuit fluidique dans un sens lors d'une étape de prélèvement d'urine dans le réservoir par l'extrémité de prélèvement et dans l'autre sens dans une étape d'injection d'urine dans la région d'analyse par l'extrémité d'injection. Notamment, la pompe peut être configurée pour inverser son sens de fonctionnement.

Dans un mode de réalisation, le circuit fluidique est linéaire, donc sans dérivation ou embranchement.

Dans un mode de réalisation, le circuit fluidique forme une boucle entre l'extrémité d'injection et l'extrémité de prélèvement.

Dans un mode de réalisation, la station est configurée pour sacrifier les premiers volumes d'urine prélevés dans le réservoir par l'extrémité de prélèvement, de sorte que la station d'urine n'injecte pas dans la région d'analyse les premiers volumes d'urine prélevés.

Dans un mode de réalisation, le circuit fluidique comprend une section de changement de sens de déplacement de l'urine, à l'intérieur de laquelle de l'urine change de sens de circulation, c'est-à-dire à l'intérieur de laquelle un front avant d'un volume d'urine devient le front arrière de ce volume d'urine.

Dans un mode de réalisation, la logique du circuit fluidique est en « dernier entré - premier sorti », LIFO, de sorte que les derniers volumes d'urine prélevés par l'extrémité de prélèvement dans le réservoir sont les premiers volumes d'urine injectés dans la région d'analyse par l'extrémité d'injection.

Dans un mode de réalisation, la station comprend un logement positionné à l'intérieur du boitier, configuré pour recevoir au moins partiellement une cartouche comprenant l'au moins une région d'analyse.

Dans un mode de réalisation, le réservoir est accessible par l'extrémité de prélèvement via un septum configuré pour être traversé par l'extrémité de prélèvement.

Cet aspect se rapporte aussi à un dispositif comprenant une station telle que décrite précédemment et une cartouche amovible de la station, dans lequel l'au moins une région d'analyse est montée sur la cartouche.

Cet aspect se rapport aussi à une méthode d'analyse d'urine à l'aide d'une station ou d'un dispositif tel que décrite précédemment, la méthode comprenant :
- une étape de prélèvement d'urine depuis le réservoir par l'extrémité de prélèvement,
- une étape d'injection d'urine dans la région d'analyse par l'extrémité d'injection.

Dans un mode de réalisation, la méthode comprend, entre l'étape de prélèvement et l'étape d'injection, une étape de précharge, au cours de laquelle de l'urine prélevée est renvoyée dans le réservoir par l'extrémité de prélèvement.

Selon un aspect, dit « sacrificiel », la description se rapporte à une station d'analyse d'urine comprenant :
- un boitier configuré pour être placé entièrement dans une cuvette de toilettes et pour recevoir un jet d'urine, le boitier étant configuré pour recevoir à l'intérieur au moins une région d'analyse apte à recevoir de l'urine pour analyse,
- un réservoir d'urine, configuré pour recevoir de l'urine issue d'une miction d'un utilisateur,
- un circuit fluidique à l'intérieur du boitier pour la circulation d'urine dans la station depuis le réservoir d'urine vers une région d'analyse, dans lequel le circuit fluidique comprend une extrémité d'injection configurée pour injecter de l'urine dans une région d'analyse et une extrémité de prélèvement configurée pour prélever de l'urine depuis le réservoir et la faire pénétrer dans le circuit fluidique,
- un analyseur, monté à l'intérieur du boitier, et configuré pour obtenir une information relative à l'urine dans la région d'analyse.

Selon cet aspect, la station est configurée pour sacrifier les premiers volumes d'urine prélevés dans le réservoir par l'extrémité de prélèvement, de sorte que station d'analyse d'urine n'injecte pas dans la région d'analyse les premiers volumes d'urine prélevés.

Dans un mode de réalisation, la station comprend en outre une extrémité de purge, la station étant configurée pour que :
- dans une position de purge, l'extrémité d'injection déverse de l'urine de l'extrémité de purge, puis,
- dans une position d'injection, l'extrémité d'injection injecte de l'urine dans la région d'analyse.

Dans un mode de réalisation, le circuit fluidique est linéaire, l'extrémité de purge étant à l'opposé de l'extrémité de prélèvement.

Dans un mode de réalisation, le circuit fluidique est configuré pour faire circuler l'urine selon une direction de déplacement unidirectionnelle, de l'extrémité de prélèvement vers l'extrémité d'injection.

Dans un mode de réalisation, l'extrémité d'injection est utilisée comme extrémité de prélèvement, par laquelle de l'urine pénètre dans le circuit fluidique, de sorte que l'extrémité d'injection est rincée par de l'urine de l'utilisateur avant l'injection.

Dans un mode de réalisation, le circuit fluidique est arrangé pour que l'extrémité d'injection est parcourue par de l'urine dans un sens lors du prélèvement et dans l'autre sens lors de l'injection.

Dans un mode de réalisation, les volumes sacrificiels sont purgés du circuit fluidique après l'étape d'injection.

Dans une mode de réalisation, les premiers volumes injectés sont les derniers volumes d'urine prélevés ou des volumes intermédiaires d'urine prélevés.

Dans un mode de réalisation, le circuit fluidique comprend une section de changement de sens de déplacement de l'urine, à l'intérieur de laquelle de l'urine change de sens de circulation, à l'intérieur de laquelle un front avant d'un volume d'urine devient le front arrière de ce volume d'urine.

Dans un mode de réalisation, la station comprend un logement positionné à l'intérieur du boitier, configuré pour recevoir au moins partiellement une cartouche comprenant l'au moins une région d'analyse.

Cet aspect se rapporte aussi à un dispositif comprenant une station telle que décrite précédemment et une cartouche amovible de la station, dans lequel l'au moins une région d'analyse est montée sur la cartouche.

Cet aspect se rapport aussi à une méthode d'analyse d'urine à l'aide d'une station ou d'un dispositif tel que décrite précédemment, la méthode comprenant :
- une étape de prélèvement d'urine depuis le réservoir par l'extrémité de prélèvement, le prélèvement comprenant des premiers volumes prélevés,
- une étape d'injection d'urine dans la région d'analyse par l'extrémité d'injection, l'injection ne comprenant pas les premiers volumes prélevés (c'est-à-dire comprenant les derniers volumes d'urine prélevés ou des volumes intermédiaires d'urine prélevés).

Dans un mode de réalisation, la méthode comprend, entre l'étape de prélèvement et l'étape d'injection, une étape de sacrifice, durant laquelle les premiers volumes prélevés sont déversés dans une extrémité de purge, notamment par l'extrémité d'injection.

Dans un mode de réalisation, la méthode comprend, après l'étape d'injection, une étape de purge durant laquelle les premiers volumes prélevés sont envoyés vers l'extrémité de purge.

Selon un aspect, dit « double-sens », la description se rapporte à une station d'analyse d'urine comprenant :
- un boitier configuré pour être placé entièrement dans une cuvette de toilettes et pour recevoir un jet d'urine, le boitier étant configuré pour recevoir à l'intérieur au moins une région d'analyse apte à recevoir de l'urine pour analyse,
- un circuit fluidique, à l'intérieur du boitier, pour la circulation d'urine dans la station vers une région d'analyse,
- un analyseur, monté à l'intérieur du boitier, et configuré pour obtenir une information relative à l'urine dans la région d'analyse.

Selon cet aspect, le circuit fluidique comprend une section de changement de sens de déplacement de l'urine, à l'intérieur de laquelle de l'urine change de sens de circulation, à l'intérieur de laquelle un front avant d'un volume d'urine devient un front arrière de ce volume d'urine.

A cet égard, la station peut comprendre une pompe configurée pour faire changer le sens du fluide dans la section de changement de sens de déplacement de l'urine. Ce changement peut se faire par inversion du sens de fonctionnement de la pompe.

Dans un mode de réalisation, la station comprend un réservoir d'urine, configuré pour recevoir de l'urine issue d'une miction d'un utilisateur, et le circuit fluidique est pour la circulation d'urine dans la station depuis le réservoir d'urine vers une région d'analyse.

Dans une mode de réalisation, le circuit fluidique comprend une extrémité d'injection configurée pour injecter de l'urine dans une région d'analyse et une extrémité de prélèvement configurée pour prélever de l'urine depuis le réservoir et la faire pénétrer dans le circuit fluidique.

Dans un mode de réalisation, la logique du circuit fluidique est en « dernier entré - premier sorti », LIFO, de sorte que les derniers volumes d'urine prélevés par l'extrémité de prélèvement dans le réservoir sont les premiers volumes d'urine injectés dans la région d'analyse par l'extrémité d'injection, de sorte que l'urine la dernière rentrée est la plus propre et celle qui est injectée.

Dans un mode de réalisation, l'extrémité d'injection est utilisée comme extrémité de prélèvement.

Dans un mode de réalisation, la section de changement de sens de direction de l'urine comprend l'extrémité d'injection, de sorte que l'extrémité d'injection est rincée par de l'urine de l'utilisateur avant l'injection.

Dans un mode de réalisation, la station comprend deux capteurs de présence de fluide disposé à deux endroits le long du circuit fluidique, dans lequel la section de changement de déplacement d'urine comprend la portion du circuit fluidique entre les deux capteurs de présence de fluide.

Dans un mode de réalisation, la station est configurée pour sacrifier les premiers volumes d'urine prélevés dans le réservoir par l'extrémité de prélèvement, de sorte que la station n'injecte pas dans la région d'analyse les premiers volumes d'urine prélevés.

Dans un mode de réalisation, le réservoir et l'au moins une région d'analyse sont agencés à proximité dans la station.

Dans un mode de réalisation, la station comprend un logement positionné à l'intérieur du boitier, configuré pour recevoir au moins partiellement une cartouche comprenant l'au moins une région d'analyse.

Cet aspect se rapporte aussi à un dispositif comprenant une station telle que décrite précédemment et une cartouche amovible de la station, dans lequel l'au moins une région d'analyse est montée sur la cartouche.

Cet aspect se rapport aussi à une méthode d'analyse d'urine à l'aide d'une station ou d'un dispositif tel que décrit précédemment, la méthode comprenant :
- une étape de prélèvement d'urine depuis le réservoir par l'extrémité de prélèvement, - une étape d'injection d'urine dans la région d'analyse par l'extrémité d'injection,
dans laquelle entre l'étape de prélèvement et l'étape d'injection, l'urine change de direction d'écoulement dans la section de changement de sens de déplacement de l'urine.

Dans un mode de réalisation, la station comprend une pompe, et le sens de fonctionnement de pompe est inversée entre l'étape de prélèvement et l'étape d'injection.

Dans un mode de réalisation, la méthode comprend, entre l'étape de prélèvement et l'étape d'injection, une étape de précharge, au cours de laquelle de l'urine prélevée est renvoyée dans le réservoir par l'extrémité de prélèvement.

Toutes ces solutions sont notamment implémentables avec les caractéristiques présentées ci-dessous, sauf si la description précise le contraire.

Le circuit fluidique possède des dimensions caractéristiques faisant que les forces de capillarité sont supérieures aux forces de gravité. Par conséquent, en l'absence de force externe autre que la gravité, l'urine ne circule pas dans le circuit fluidique.

Dans un mode de réalisation, la station comprend un logement positionné à l'intérieur du boitier, configuré pour recevoir au moins partiellement une cartouche comprenant l'au moins une région d'analyse. La cartouche peut comprendre une pluralité de régions d'analyses, configurées pour être chacun sélectivement positionnée au regard de l'extrémité d'injection.

Dans une variante, le logement comprend une forme annulaire et la cartouche est mobile en rotation dans la station.

La description se rapporte aussi à un dispositif comprenant une station telle que décrite ici, et une cartouche, dans lequel l'au moins une région d'analyse est montée sur une cartouche amovible de la station.

La station peut comprendre un orifice de collecte positionné sur le boitier et l'extrémité de prélèvement prélève du réservoir via l'orifice de collecte.

Le réservoir peut est formé en partie par le boitier. Le réservoir est, dans un mode de réalisation, à l'extérieur du boitier, afin de recevoir de l'urine qui coule sur le boitier. A cet égard, le réservoir est un réservoir tampon (l'urine y passe et y est prélevée par l'extrémité de prélèvement) ou un réservoir de stockage où l'urine s'accumule.

Dans un mode de réalisation, le réservoir est accessible par l'extrémité de prélèvement via un septum configuré pour être traversé par l'extrémité de prélèvement.

La station comprend typiquement une pompe configurée pour faire circuler le fluide dans le circuit fluidique, par exemple pour le faire circuler dans un sens ou dans l'autre. La pompe peut être une pompe péristaltique, ce qui garder l'urine dans de la tuyauterie.

Dans un mode de réalisation, l'extrémité de prélèvement comprend une aiguille. L'aiguille peut être biseautée, notamment pour percer le septum et pour percer la chambre des cartouches.

Dans un mode de réalisation, l'extrémité d'injection est mobile à l'intérieur du boitier, par exemple en translation, grâce à un moteur.

Dans un mode de réalisation, le réservoir et l'au moins une région d'analyse sont agencés à proximité (lorsque ladite région d'analyse est positionnée pour l'étape d'injection), par exemple dans l'alignement de l'axe de translation de l'extrémité d'injection. Alternativement, l'extrémité de purge et l'au moins une région d'analyse sont agencés à proximité (lorsque ladite région d'analyse est positionnée pour l'étape d'injection), par exemple dans l'alignement de l'axe de translation de l'extrémité d'injection.

Dans un mode de réalisation, l'extrémité de purge est à fluidiquement à l'opposé de l'extrémité d'injection.

Dans un mode de réalisation, le circuit fluidique est linéaire, c'est-à-dire sans dérivation ou embranchement.

Dans un mode de réalisation, l'extrémité d'injection est mobile entre notamment une position d'injection, durant laquelle l'extrémité d'injection injecte de l'urine dans la région d'analyse, et une position de prélèvement, durant laquelle l'extrémité d'injection prélève de l'urine dans le réservoir. Selon les variantes, l'extrémité d'injection peut être mobile entre une position d'injection et une position de purge, durant laquelle l'extrémité d'injection déverse de l'urine vers une extrémité de purge.

Dans un mode de réalisation, la station est configurée pour mettre en oeuvre une étape de précharge, entre l'étape de prélèvement et l'étape d'injection, au cours de laquelle les derniers volumes d'urine prélevés sont réinjectés dans le réservoir. L'étape de précharge peut être mise en oeuvre en réponse à une détermination qu'une section de référence entre les deux capteurs de présence de fluide ont indiqué que la section de référence ne contenait que de l'urine.

La station comprend une circuiterie de contrôle, avec un processeur et une mémoire apte à stocker un programme que le processeur peut exécuter. La circuiterie de contrôle est notamment configurée pour contrôler la position de l'extrémité d'injection (entre les différentes positions) à contrôler la pompe et apte à contrôler la position de l'au moins une région d'analyse dans le boitier.

L'analyseur comprend typiquement un analyseur optique, par exemple un capteur CCD ou une caméra.

Dans un mode de réalisation, la station comprend un logement positionné à l'intérieur du boitier, configuré pour recevoir au moins partiellement une cartouche comprenant l'au moins une région d'analyse. Le logement peut avoir une forme annulaire. La cartouche est typiquement mobile en rotation.

La description peut aussi se rapporter à un dispositif d'analyse d'urine comprenant une station telle que décrite précédemment et une cartouche telle que décrite précédemment.

### Présentation des figures

D'autres caractéristiques, détails et avantages apparaîtront à la lecture de la description détaillée ci-après, et à l'analyse des dessins annexés, sur lesquels :
- [FIG. 1] : la figure 1 présente une représentation schématique et simplifiée d'un dispositif d'analyse d'urine installé dans une cuvette de toilettes,
- [FIG. 2] : la figure 2 présente une vue éclatée du dispositif d'analyse d'urine, dans laquelle la station et la cartouche sont visibles,
- [FIG. 3] : la figure 3 présente une vue détaillée d'une cartouche selon un mode de réalisation,
- [FIG. 4] : la figure 4 présente une vue en coupe d'une cartouche et d'une station selon un mode de réalisation, à l'emplacement d'un analyseur optique de la station,
- [FIG. 5] : la figure 5 présente deux modes de réalisation d'une vue en coupe du dispositif (station et cartouche) au niveau de l'extrémité d'injection,
- [FIG. 6] : la figure 6 présente des vues schématiques du circuit fluidique d'une station de mesure conforme à un mode de réalisation dit « sacrificiel »,
- [FIG. 7] : la figure 7 présente une vue plus réaliste d'un circuit fluidique similaire à celui de la figure 6,
- [FIG. 8] : la figure 8 présente des vues schématiques du circuit fluidique d'une station de mesure conforme un mode de réalisation dit « injecteur - préleveur »,
- [FIG. 9] : la figure 9 présente des vues schématiques du circuit fluidique d'une station de mesure conforme à des modes de réalisation dit « sacrificiel » et « injecteur - préleveur »,
- [FIG. 10] : la figure 10 présente des vues schématiques du circuit fluidique d'une station de mesure conforme à un mode de réalisation dit « doubles sens » (et approprié pour le mode de réalisation « sacrificiel »),
- [FIG. 11] : la figure 11 présente des vues schématiques du circuit fluidique d'une station de mesure conforme aux des modes de réalisation dit « injecteur-préleveur », « double-sens » et approprié pour le mode de réalisation « sacrificiel »),
- [FIG. 12] : la figure 12 présente une vue plus réaliste d'un circuit fluidique similaire à celui de la figure 11,
- [FIG. 13] : la figure 13 présente une vue simplifiée du circuit fluidique de la figure 12, dans une étape de prélèvement,
- [FIG. 14] : la figure 14 présente une vue simplifiée du circuit fluidique de la figure 12, dans une étape d'injection,
- [FIG. 15] : la figure 15 présente une vue simplifiée du circuit fluidique de la figure 12, dans une étape de purge,
- [FIG. 16] : la figure 16 présente une architecture schématique d'une station de mesure et son écosystème.

### Description détaillée

La présente description présente différentes architectures de circuits fluidiques d'un dispositif d'analyse d'urine comprenant un boitier dimensionné pour être disposé sur une paroi d'une cuvette de toilette. Les documents suivants décrivent un exemple d'un tel dispositif d'analyse : WO2021175909 et WO2021175944, WO2023036805, WO2023036806, WO2023036808, WO2023036809. On parlera par la suite de documents WO pour les désigner d'une façon générale.

Le circuit fluidique a pour fonction d'acheminer de l'urine collecté par le boitier vers une région d'analyse, qui comprend typiquement un réactif, positionnée à l'intérieur du boitier, et notamment vers une cartouche amovible reçu dans le boitier.

Plusieurs modes de réalisations et variantes de circuits fluidiques vont être présentés en relation avec un dispositif conforme aux documents WO précités à titre d'exemple.

### Orientation

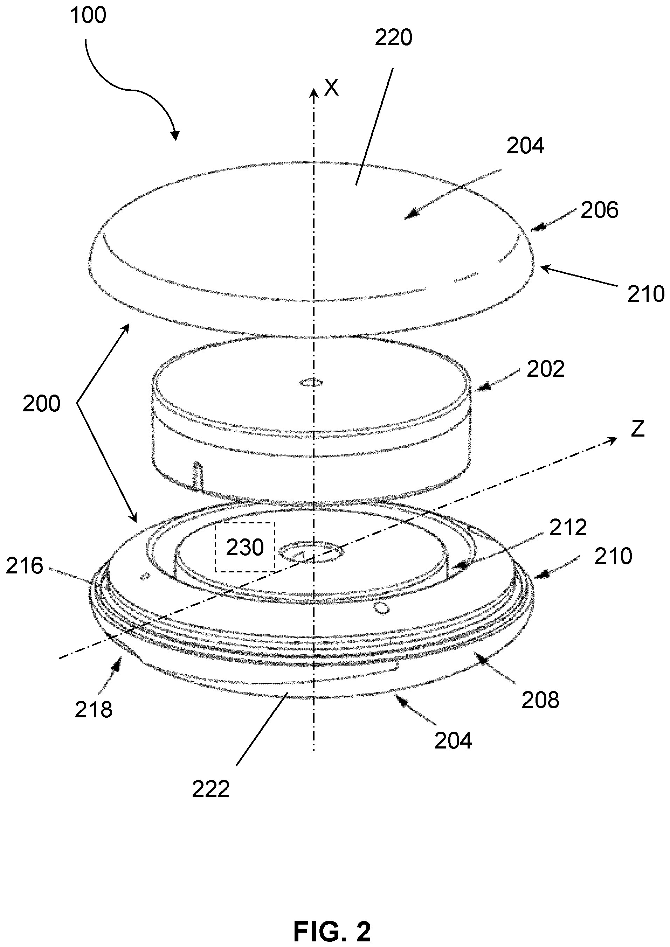

Dans la suite de l'exposé, les notions de "haut" et "bas", "supérieur" et "inférieur", etc. sont définies par rapport à une direction Z, telle que définie sur la figure 2. Le haut selon la direction Z est défini dans une position d'utilisation normale du dispositif d'analyse d'urine fixé dans la cuvette des toilettes.

### Forme générale du boitier

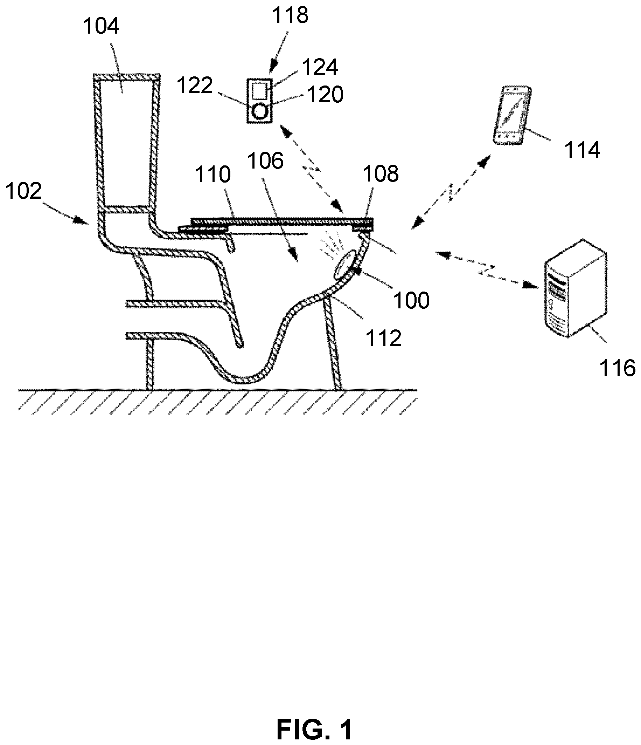

La figure 1 illustre schématiquement un dispositif d'analyse 100 (appelé aussi "dispositif 100" par la suite) pour l'analyse d'urine installé dans les toilettes 102. Les toilettes 102 comprennent généralement un réservoir d'eau 104, une cuvette 106, un siège 108 et un couvercle de siège 110. Le dispositif d'analyse 100 est configuré pour être placé entièrement dans la cuvette des toilettes. Par "dans la cuvette", on entend "placé dans le volume intérieur défini par la cuvette". Le dispositif d'analyse 100 est disposé de manière amovible dans les toilettes 102. Par exemple, le dispositif d'analyse 100 peut être facilement retiré des toilettes pour remplacer une cartouche, puis replacé dans les toilettes 102. Le dispositif d'analyse 100 est placé sur une paroi 112 de la cuvette 106 des toilettes. Le dispositif d'analyse 100 est placé de telle sorte qu'il se trouve généralement sous le jet d'urine d'un utilisateur, de sorte que lorsqu'un utilisateur urine (généralement en position assise), l'urine entre en contact avec le dispositif d'analyse 100. Le dispositif d'analyse 100 peut communiquer à distance avec une entité distante, telle que le smartphone 114 ou un serveur 116.

Comme l'illustre plus en détail la figure 2, le dispositif d'analyse 100 peut comprendre une station d'analyse d'urine 200 (appelée aussi « station 200 » par la suite) et une cartouche 202, montée de manière amovible sur la station 200. La cartouche 202 comprend du réactif apte à réagir avec de l'urine (appelé « réactif urinaire »). Dans un mode de réalisation sans cartouche 202, le dispositif d'analyse 100 et la station d'analyse 200 sont confondus.

Alternativement, la station 200 comprend directement du réactif urinaire sans partie amovible. Alternativement, la station 200 peut se recharger en versant du réactif sous forme liquide.

Alternativement, l'analyse d'urine se fait sans réactif. On peut citer à ce titre des analyses optiques, de type spectroscopie.

La station 200 peut comprendre un boitier 204 qui peut comporter deux coques, notamment une coque avant 206 et une coque arrière 208. La coque avant 206 et la coque arrière 208 peuvent coopérer l'une avec l'autre par l'intermédiaire d'un mécanisme de fixation 216, dans un plan normal à l'axe X. La coque avant 206 et la coque arrière 208 peuvent être assemblées de manière réversible, par exemple par vissage ou par clipsage. Dans une variante, la coque avant 206 et la coque arrière 208 peuvent être assemblées de manière permanente, par exemple par collage, clipsage, magnétisation, ou soudage par ultrasons. D'autre moyens de fixation peuvent être utilisés pour assembler les deux coques.

Le boitier 204 est étanche. Seuls un orifice de collecte et un orifice de purge permettent le passage d'urine entre l'intérieur et l'extérieur. Ces orifices seront décrits plus en détails par la suite.

Comme on peut le voir sur les figures, le boitier 204 peut avoir une forme globale extérieure de galet circulaire. En d'autres termes, le boitier 204 présente une forme sphéroïde. L'axe X est la ligne centrale du boitier. Avantageusement, la coque avant 206 peut être sensiblement symétrique en rotation, ce qui donne un aspect aérodynamique au dispositif une fois installé. Le boitier 204 sert de collecteur d'urine.

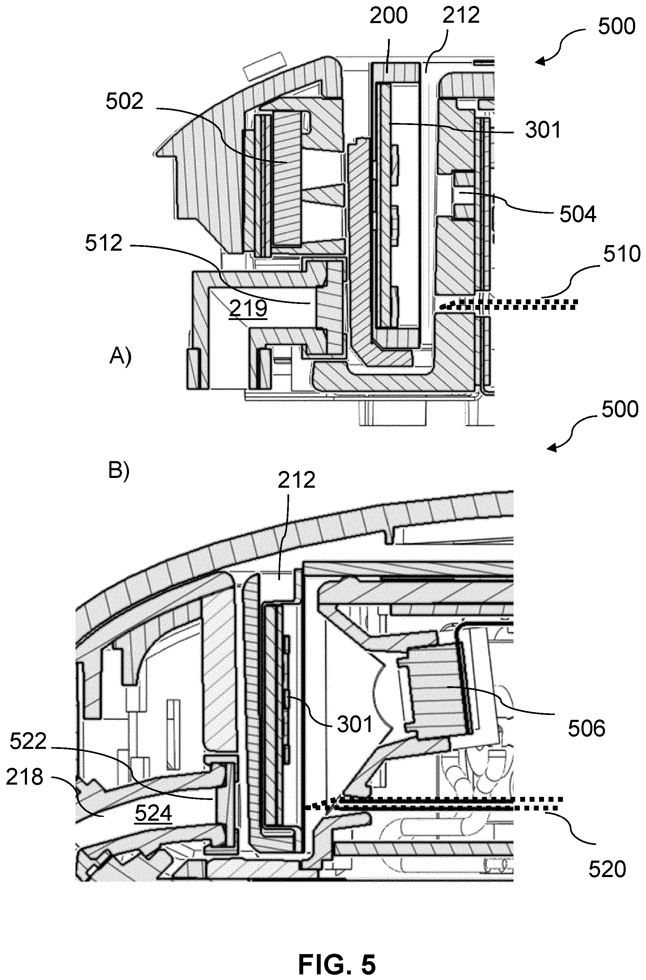

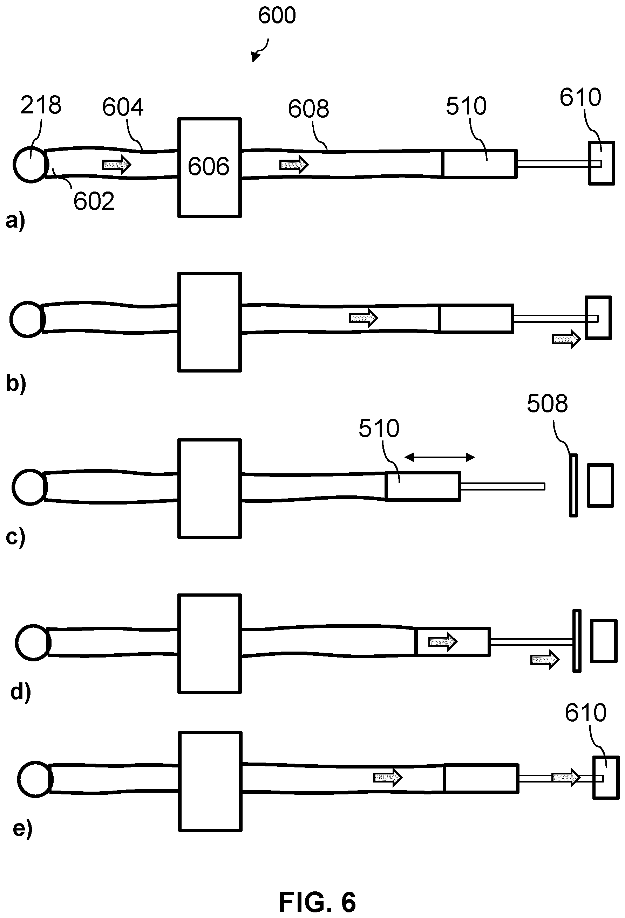

Le boitier 204 comprend une face avant 220 pour recevoir un flux d'urine directement d'un utilisateur urinant sur les toilettes et une face arrière 222 opposée à la face avant 220. Comme illustré à la figure 2, la face avant 220 peut être disposée sur la coque avant 206 et la face arrière 222 peut être disposée sur la coque arrière 208. La face avant 220 est orientée vers l'intérieur de la cuvette 106. La face avant 220 est donc destinée à recevoir l'urine lorsque l'utilisateur urine en s'asseyant sur les toilettes 102. Comme le montrent les figures 5 et 6, la face arrière 222 fait face à la paroi intérieure 112 de la cuvette 106. Par la suite de la description, il est entendu par un objet faisant face à la paroi de la cuvette, un objet faisant face à la paroi de la cuvette la plus proche de l'objet en question, et non la paroi de la cuvette en vis-à-vis de l'autre côté du volume interne de la cuvette.

La face avant 220 et la face arrière 222 comportent chacune un bord incurvé 210. Les bords incurvés 210 respectifs de la face avant et de la face arrière se rejoignent au niveau d'une zone de jonction équatoriale. Ainsi, la surface extérieure du boitier 204, constituée de la face avant 220 et de la face arrière 222, est définie par des lignes courbes et forme un objet généralement convexe.

La surface extérieure du boitier 204 peut également être blanche ou de couleur claire. La couleur de la surface extérieure peut être similaire à celle des toilettes, ce qui accroît la discrétion du dispositif.

Le boitier 204 peut avoir un diamètre, mesuré dans la direction orthogonale à l'axe X, compris entre 50 mm et 150 mm. Le boitier 204 peut avoir une épaisseur, mesurée dans la direction de l'axe X, comprise entre 15 mm et 50 mm. Ainsi, le boitier 204 est suffisamment compact pour être entièrement logé dans la cuvette des toilettes. Le dispositif d'analyse d'urine 100 est discret. De plus, le boitier 204 est suffisamment grand pour entrer systématiquement en contact avec l'urine reçue dans la cuvette. L'utilisateur peut alors uriner dans les toilettes sans se soucier du dispositif d'analyse d'urine, ou alternativement viser sommairement.

Selon un autre aspect, dans un mode de réalisation, le boitier 204 présente un facteur de forme général tel que le rapport entre l'épaisseur et le diamètre est compris entre 0,2 et 0,5, et même de préférence entre 0,3 et 0,4. De telles proportions rappellent un galet naturel et donnent au dispositif un aspect apaisant.

De préférence, le boitier 204 est constitué d'un matériau hydrophile. Par exemple, le matériau du boitier 204 peut être : une céramique, un polyamide (PA), un silicone ou un polymère hydrophile. La surface extérieure du boitier 204 peut également être traitée avec un traitement de surface hydrophile, par exemple acuWet^{®} d'Aculon, un polymère hydrophile, ou Pebax^{®} d'Arkema.

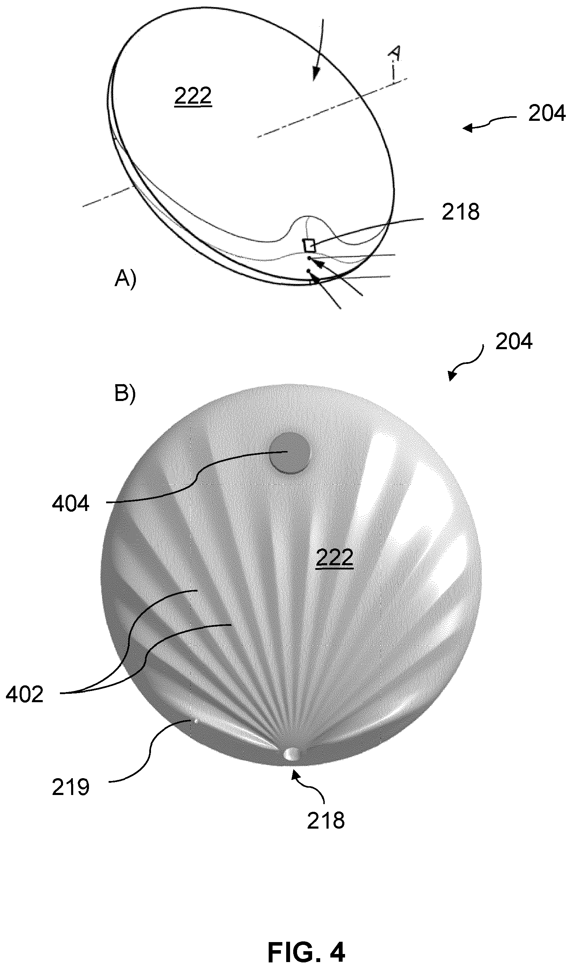

La station 200 comprend un orifice de collecte 218, située par exemple sur la coque arrière 208. Comme cela sera expliqué plus en détail ci-dessous, l'orifice de collecte 218 est configurée pour collecter l'urine s'écoulant sur la surface du boitier 204. La station 200 comprend également une purge, dont l'orifice de purge 219 est visible notamment sur les figures 4 B) et 5 A), configurés pour drainer le liquide hors du dispositif 100.

### Logement pour cartouche

La station 200 comprend typiquement un compartiment annulaire 212, situé à l'intérieur du boitier 204, disposé autour d'un axe de rotation X. Le compartiment annulaire 212 est configuré pour recevoir au moins partiellement la cartouche 202 montée de manière rotative autour de l'axe de rotation X (une fois en position dans le compartiment annulaire 212). La cartouche comprend une pluralité de régions d'analyse.

Dans le mode de réalisation des figures, la cartouche 202 comprend du réactif urinaire, notamment au moyen d'une pluralité de supports de test qui comprennent chacun au moins un réactif urinaire, par exemple un réactif sec. Dans l'exemple illustré, la pluralité de supports de test est disposée le long d'un cercle ou d'un arc de cercle autour de l'axe de rotation X et forment la pluralité de régions d'analyse. Dans un mode de réalisation, les supports de test sont des bandelettes de test. Les supports de test peuvent être enfermés, par exemple individuellement, dans une chambre étanche.

Alternativement, la cartouche 202 comprend des chambres servant de volumes indépendants pour recevoir de l'urine pour subir une analyse optique de l'urine directement. Les chambres forment alors la pluralité de régions d'analyse.

Le compartiment annulaire 212 s'étend typiquement sur 360° et forme une gorge configurée pour recevoir au moins partiellement la cartouche 202.

Le document EP4338839 décrit une méthode pour obtenir des chambres étanches dans une cartouche.

Dans un mode de réalisation, toutes les régions d'analyse se trouvent au même endroit dans le boitier pour recevoir de l'urine et/ou être analysée (comme illustré, avec une rotation de la cartouche). Alternativement (non illustré), les régions d'analyse peuvent être à des endroits différents à l'intérieur du boitier pour recevoir de l'urine et/ou être analysée.

### Ensemble de test

Un ensemble de test est disposé à l'intérieur du boitier 204 et configuré pour effectuer une analyse sur l'urine collectée par l'orifice de collecte.

L'ensemble de test comprend notamment un circuit fluidique, configuré pour notamment acheminer de l'urine depuis un réservoir d'urine jusqu'à l'au moins une région d'analyse. Le réservoir d'urine est typiquement formé par le boitier. L'urine pénètre ensuite par l'orifice de collecte pour pénétrer dans le circuit fluidique. Dans un mode de réalisation qui sera décrit par la suite, l'orifice de collecte est obstrué par un septum qui peut être traversé par une aiguille. Le circuit fluidique comprend une extrémité d'injection (aussi appelé injecteur) et l'orifice de collecte. La station 200 comprend en outre une pompe (ou un système de pompe) pour mettre le fluide en mouvement dans le circuit fluidique. La pompe peut être une pompe péristaltique, ce qui garder l'urine dans de la tuyauterie (plus pratique pour le nettoyage et pour éviter la cross-contamination).

L'ensemble de test comprend en outre un analyseur 230. La pompe aspire l'urine au travers de l'orifice de collecte 218, puis l'extrémité d'injection injecte l'urine dans une région d'analyse, par exemple sur du réactif urinaire ou dans une chambre. L'extrémité d'injection est ainsi configurée pour déposer l'urine dans la région d'analyse. Dans un mode de réalisation, la région d'analyse peut être amenée, par exemple par rotation de la cartouche, en vis-à-vis de l'analyseur 230 pour effectuer l'analyse. Enfin, l'analyseur mesure certaines valeurs de propriétés (par exemple, des propriétés physiques/chimiques, telles que la couleur) du réactif après qu'il est entré en contact avec l'urine, ou bien de l'urine directement. Dans un cas, l'analyseur est un analyseur optique (par exemple une caméra) configuré pour analyser les propriétés optiques du réactif. Dans d'autre modes de réalisation, l'analyse optique peut être configuré pour effecteur une spectroscopie de l'urine. Par conséquent, l'analyseur est configuré pour obtenir une information relative à l'urine dans la région d'analyse, que ce soit une information directement obtenue à partir de l'urine ou une information indirectement obtenue à partir de l'urine (via le réactif).

L'extrémité d'injection et la cartouche peuvent se déplacer l'un par rapport à l'autre, notamment pour pouvoir sélectionner une région d'analyse (par exemple un réactif) qui doit recevoir de l'urine de l'extrémité d'injection et pour que l'extrémité d'injection puisse ouvrir (par exemple, percer) la chambre, par exemple à l'aide d'une aiguille ou d'un dispositif semblable à une aiguille. Les documents WO cités précédemment détaillent le déplacement de l'extrémité d'injection (appelé injecteur ou seringue dans les documents WO).

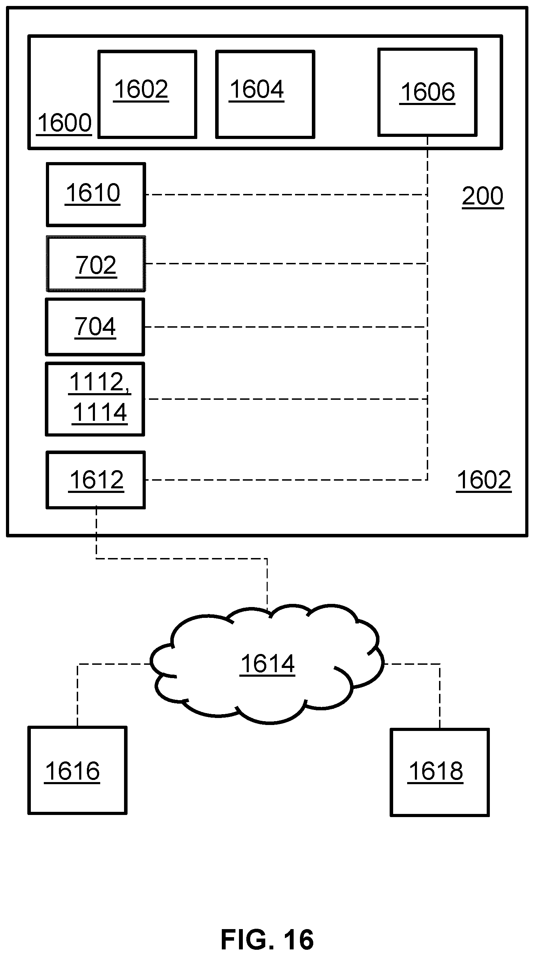

La station 200 comprend une circuiterie de contrôle 1600, illustrée en figure 16, apte à contrôler les différents composants du dispositif 100, tels que la position de l'extrémité d'injection ou encore l'activation de la pompe ou encore, le cas échéant, de valve.

### Cartouche

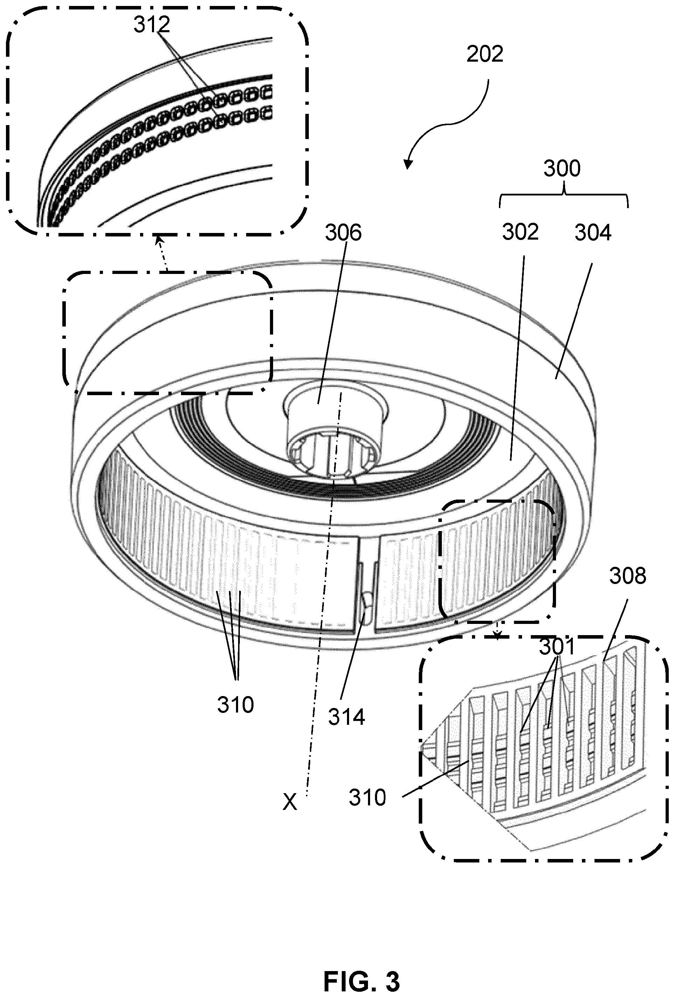

La figure 3 montre une vue éclatée d'une cartouche 202. La cartouche 202 comprend au moins une région d'analyse, par exemple au moins un support de test 301, notamment plusieurs zones de réaction (notamment plusieurs supports de test 301) configurés pour recevoir l'urine de l'injecteur. Dans un mode de réalisation, chaque support de test 301 contient un réactif urinaire qui réagit de manière spécifique au contact de l'urine.

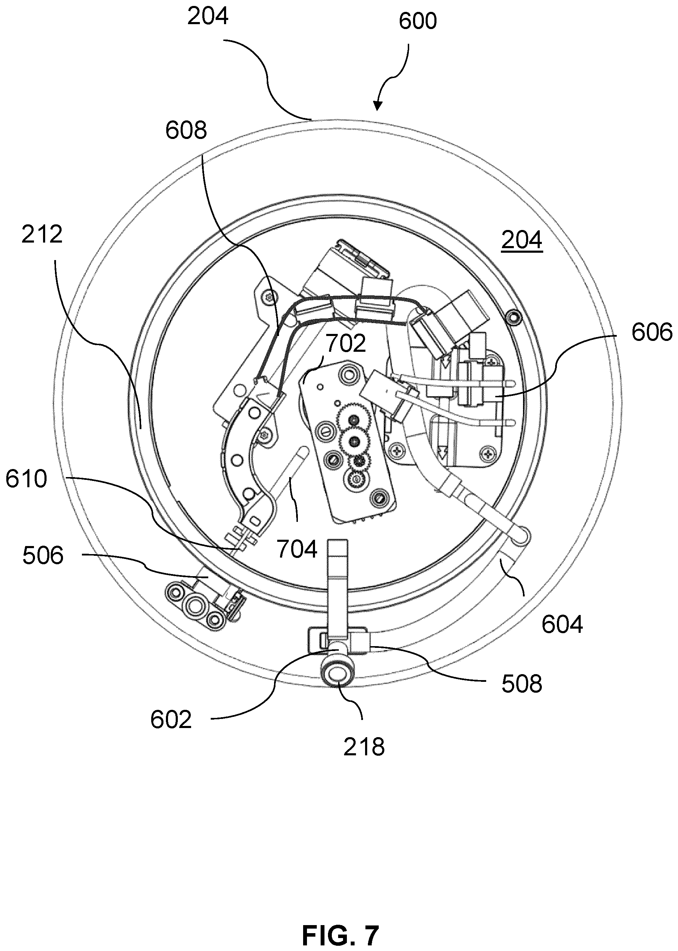

La cartouche 202 comprend un support rotatif 300, configuré pour être entraîné en rotation par la station 200, par exemple par un moteur (référence 702 en figure 7). Dans le cadre d'une utilisation normale de la cartouche 202 et du dispositif 100, les zones de réaction (les supports de test 301 donc) restent fixés au support rotatif et ne se déplacent pas par rapport à ce dernier.

Dans une réalisation, le support rotatif 300 a une forme de cylindre circulaire droit d'au moins 80% d'une forme de cylindre creux s'étendant annulairement autour d'un axe qui est, lorsque la cartouche 202 est montée dans la station 200, l'axe de rotation X. Chaque support de test 301 peut être une bandelette de test. Le support rotatif 300 peut comprendre une partie annulaire 302 et une partie cylindrique 304, qui s'étend à partir d'une extrémité radiale extérieure de la partie annulaire 302. La partie cylindrique 304, lorsqu'elle est utilisée, est logée à l'intérieur du compartiment annulaire 212. Les supports de test 301 sont positionnés le long de la portion cylindrique 304, de manière à pouvoir défiler sélectivement et/ou successivement devant l'injecteur et l'analyseur. Par exemple, les supports de test 301 font partie d'un support 308, qui comprend plusieurs chambres 310, séparées les unes des autres le long d'un périmètre autour de l'axe X. Au moins une bandelette de test est reçue dans une chambre 310.

Les chambres 310 sont disposées les unes à côté des autres en forme de cylindre circulaire droit d'au moins 80 % du cercle. Pour permettre à la lumière de passer, le support 308 comprend au moins une ouverture 312 par chambre 310 (représentée dans le zoom supérieur gauche où le support rotatif est représenté comme transparent). Les chambres 310 sont toutes à égale distance de l'axe de rotation X, de sorte que l'injecteur peut injecter sélectivement l'urine une fois que la chambre souhaitée est positionnée à l'endroit voulu face à l'injecteur. L'injecteur peut se déplacer vers la chambre 310, par exemple à l'aide d'un moteur (référencé 704 sur la figure 7) et percer un couvercle fermant la chambre 310 (visible sur la figure 4). Une ouverture de purge 314 est prévue dans le support rotatif 300 pour permettre l'évacuation de l'urine de l'injecteur dans le circuit de purge, et donc vers l'extérieur du dispositif 100, via l'orifice de purge 219 disposée sur le boitier 204.

La partie annulaire 302 du support rotatif 300 reste à l'extérieur du compartiment annulaire 212 pour renforcer la partie cylindrique et/ou entraîner en rotation la cartouche 202. À cette fin, la partie annulaire 302 peut comprendre un accouplement mécanique 306, qui coopère avec un arbre de la station 200.

Les dimensions relatives à la cartouche 202 sont divulguées dans les documents précités. La dimension maximale du dispositif 100 transversalement à l'axe de rotation X est inférieure à 15 cm, voire inférieure à 10 cm. La dimension maximale du dispositif le long de l'axe de rotation X est inférieure à 5 cm.

### Orifice de collecte

L'orifice de collecte 218 est configuré pour recevoir l'urine qui s'écoule par gravité sur la surface extérieure du boitier 204. L'urine est collectée directement sur la face avant 220 et la face arrière 222 du boitier 204.

L'orifice de collecte 218 est une ouverture configurée pour collecter le liquide, de sorte que le liquide puisse pénétrer dans le dispositif d'analyse d'urine. L'orifice de collecte 218 est généralement circulaire, avec un diamètre de préférence compris entre 0,3 mm et 2 mm. Le diamètre de l'orifice de collecte peut être choisi pour maximiser le volume d'urine collecté sur la surface extérieure du boitier 204.

Comme on peut le voir sur les figures, l'orifice de collecte 218 est situé sur la face arrière 222. Ainsi, l'orifice de collecte 218 fait face à la paroi interne 112 des toilettes lorsque le dispositif d'analyse d'urine 100 est positionné dans les toilettes. Cette position permet à l'orifice de collecte 218 d'être cachée à la vue de l'utilisateur par la face avant 220 du boitier. La face avant 220 visible par l'utilisateur ressemble à un simple galet uniforme, comme déjà mentionné, sans points singuliers ni trous. Il convient également de noter que cette position empêche l'introduction de contaminants ou d'éléments qui pourraient obstruer le circuit fluidique.

L'orifice de collecte 218 est situé sur une partie inférieure de la face arrière 222. Par "sur une partie inférieure de la face arrière", on entend "sur le dernier quart de la face selon la direction Z à partir de l'extrémité inférieure du boitier 204". L'extrémité inférieure fait face au fond de la cuvette 106 lorsque le boitier 204 est positionné dans la cuvette. L'extrémité inférieure se situe à l'opposé du sommet 550. Cette position correspond à une position normale d'utilisation. Cette position permet à l'urine d'être collectée par gravité sur la majeure partie de la surface extérieure du boitier 204.

La figure 4 illustre deux variantes de la face arrière 222 avec l'orifice de collecte 218. Selon une première variante représentée sur la figure 4 A), qui est décrite en détail dans le document WO2021175944, la face arrière 222 est lisse avec une géométrie particulière pour récupérer de l'urine, notamment un renfoncement en partie basse du boitier. Selon une deuxième variante représentée sur la figure 4 B), qui est décrite en détail dans le document EP23192264 (numéro de dépôt), la face arrière 222 présente un réseau de nervures qui permettent de diriger des flux d'urine vers l'orifice de collecte 218.

En particulier, la distance séparant l'orifice de collecte 218 d'un bord inférieur du boitier 204 est inférieure à 40 mm, de préférence inférieure à 20 mm. Comme illustré, selon un mode de réalisation particulier, l'orifice de collecte 218 est disposée à quelques millimètres au-dessus du bord inférieur du boitier 204. En variante, l'orifice de collecte 218 peut se trouver sur le bord inférieur (le bord inférieur étant défini lorsque le dispositif 100 est placé pour être utilisé dans les toilettes).

L'orifice de collecte 218 peut être couvert par un filtre à mailles. Le filtre à mailles est par exemple de forme oblongue et recouvre l'orifice de collecte 218. L'ouverture moyenne des mailles du filtre est, par exemple, de 20 microns. Le filtre à maille empêche l'introduction de contaminants ou d'éléments susceptibles d'obstruer le circuit fluidique et filtre l'urine reçue dans l'orifice de collecte 218. La maille du filtre peut être en métal.

### Attache

Le boitier 204 peut être maintenu en position dans la cuvette grâce à une fixation, dont la figure 4 B) représente une partie avec un plot en saillie 404 configuré pour coopérer avec un bras qui s'attache au rebord de la cuvette. Alternativement, une liaison magnétique ou autre est possible.

### L'analyseur

La figure 5, qui représente deux vues en coupe de deux modes de réalisation A) et B) différents (échelle entre les deux modes de réalisation non respectée), illustre notamment l'interaction entre la cartouche 202 et la station 200 au niveau de l'analyseur 500, avec deux modes de réalisation de l'analyseur. L'architecture fluidique est en outre différente entre les modes de réalisation A) et B), mais cela est indépendant de l'analyseur.

L'analyseur 500de la figure 5 A) comprend une source lumineuse 502 (par exemple, deux sources lumineuses) et au moins un capteur optique 504 sous la forme ici d'un capteur CCD ("*Charge Couple Device"* en anglais ou Dispositif de couplage de charge en français). La lumière va de la source lumineuse 502 au capteur optique 504 en traversant la cartouche 202 et en particulier la partie cylindrique 304, l'ouverture 312 du support 308, le support de test 301 et donc le réactif urinaire sur le support de test 301.

Dans un mode de réalisation, l'analyseur 500 est configuré pour mesurer l'absorbance d'une partie des supports de test 301 (notamment la ligne de test et/ou la ligne de contrôle d'une bandelette comme cela sera expliqué plus loin). L'absorbance est détectée par la source lumineuse (par exemple une LED) qui peut faire passer de la lumière à travers la bande, et le capteur optique qui reçoit le spectre avec une dizaine de longueurs d'onde.

L'analyseur 500 de la figure 5 B) comprend un capteur optique sous la forme d'une caméra 506 capable de détecter un changement de couleur, notamment un changement d'intensité de couleur du réactif, et donc ici d'une partie des supports de test 301 (notamment la ligne de test et/ou la ligne de contrôle d'une bandelette). La caméra peut détecter une couleur en valeurs RVB par exemple. Une source lumineuse peut être prévue pour que la caméra 406 puisse mieux identifier les couleurs.

La figure 5 B) illustre notamment le réservoir mentionné précédemment et référencée 524. Le réservoir 524 est ici formé par le boitier 204 : l'ouverture 218 est allongée pour créer un volume de stockage, qui est le réservoir. L'orifice de collecte 218, à l'extrémité du réservoir 522 du côté intérieur du boitier, est fermée par un septum 522. Une grille (non visible), peut être positionnée à l'entrée du réservoir pour filtrer le débris.

Le réservoir est à l'extérieur du boitier 204, afin de pouvoir collecter librement l'urine coulant sur le boitier. Le réservoir communique donc avec l'extérieur de la station, et sélectivement avec l'intérieur (via l'orifice de collecte).

### L'extrémité d'injection

La figure 5 illustre aussi une extrémité d'injection 510 sur la figure 5 A) et une extrémité d'injection 520 sur la figure 5 B). Des références numériques différentes sont utilisées car les circuits fluidiques associés peuvent être différents. Cela sera décrit en détail par la suite.

L'extrémité d'injection 510, 520 peut comprendre une aiguille, notamment biseautée, pour par exemple pouvoir percer les chambres étanches et/ou traverser le septum

L'extrémité d'injection 510, 520 peut être déplacée entre plusieurs positions. On distingue notamment une position neutre durant laquelle l'extrémité d'injection ne coopère ni avec le réservoir, ni le circuit de purge, ni la région d'analyse (donc notamment ne traverse pas le logement 212), une position d'injection durant laquelle l'extrémité d'injection pénètre dans le logement 212 pour injecter de l'urine sur une région d'analyse 508, et une troisième position, dont la fonction dépend du circuit fluidique et qui sera décrit plus tard : la troisième position peut être une position de prélèvement, une position de purge ou une position de sacrifice (cette position étant identique à la position de purge).

En position neutre, l'extrémité d'injection 510, 520 est, dans le mode de réalisation illustré sur les figures, située radialement à l'intérieur du logement 212. Cela permet de maximiser le rayon du compartiment annulaire tout en minimisant la taille de la station 200.

### Circuit fluidique

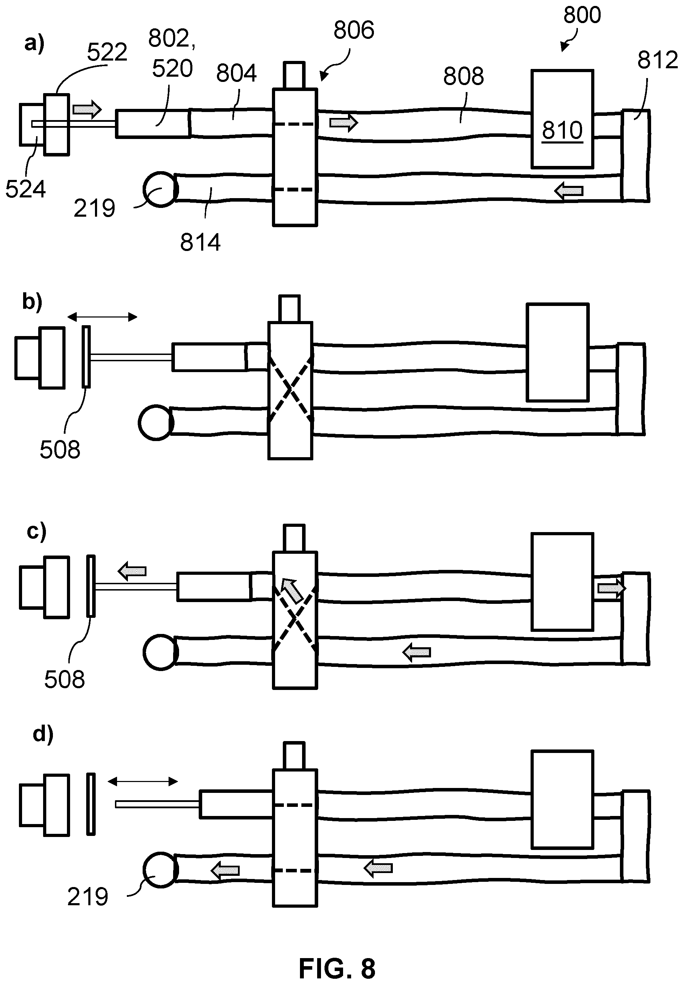

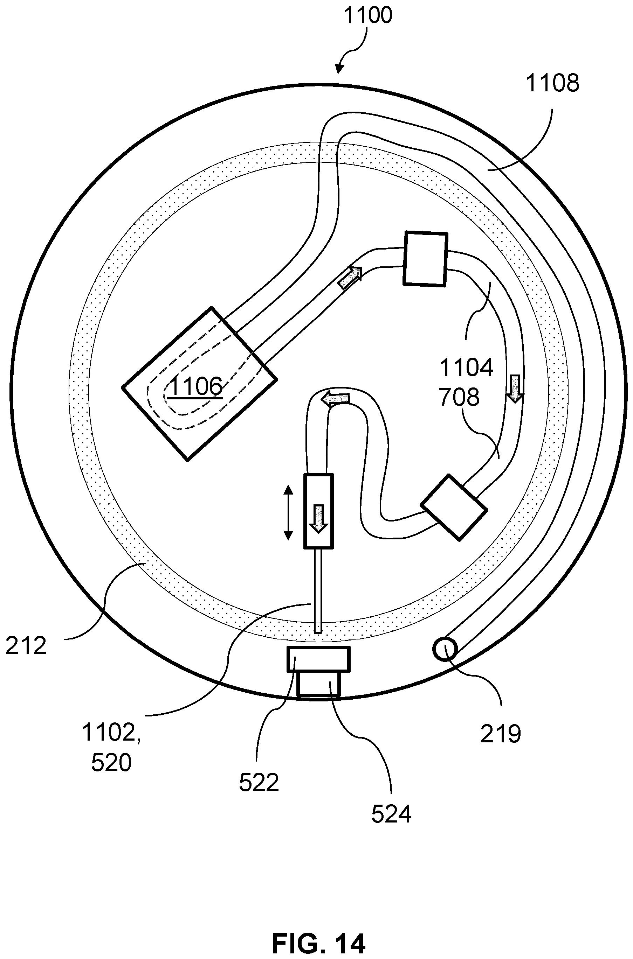

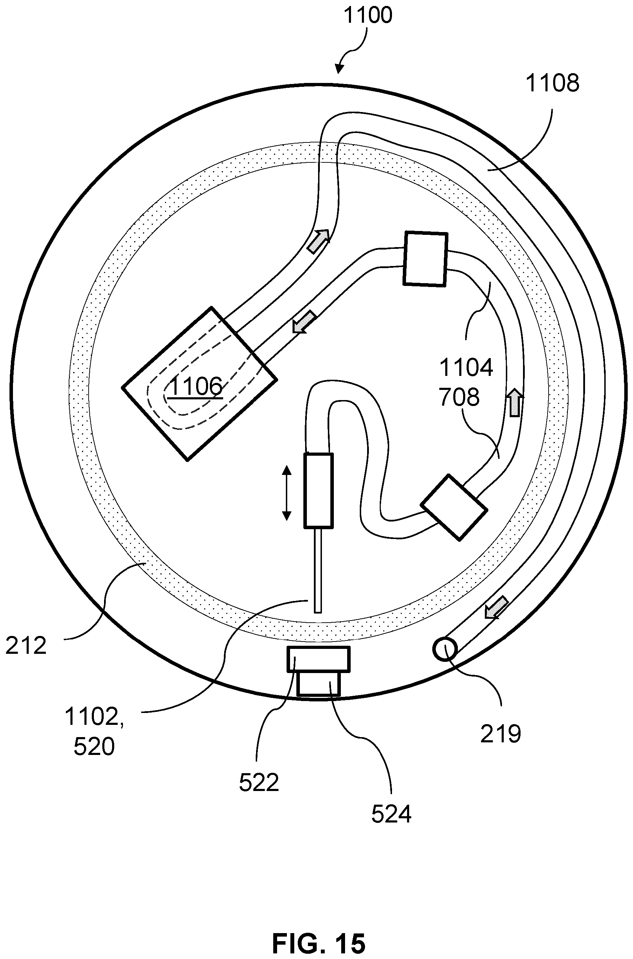

Pour acheminer l'urine qui ruisselle sur le boitier 204 jusqu'à la région d'analyse 508, la station d'analyse d'urine comprend un circuit fluidique dont plusieurs modes de réalisation ou variantes sont représentées en figures 6 à 15. Notamment, deux modes de réalisation particuliers sont illustrés en figures 6 et 7 (les cartouches 202 ne sont pas représentées pour simplifier les figures) et d'autresmodes de réalisation sont illustrés en figure 8 à 15. Des références numériques différentes sont utilisées car les circuits fluidiques sont différents.

Les circuits fluidiques présentés comprennent tous un réservoir, une extrémité de prélèvement, qui prélève de l'urine dans le réservoir en passant par l'orifice de collecte 218, de la tuyauterie, une extrémité d'injection 510, 520, et une extrémité de purge, qui comprend l'orifice de purge 219. L'extrémité de purge comprend typiquement de la tuyauterie reliée à l'orifice de purge. L'extrémité de purge sert à mettre du fluide à l'écart dans le circuit fluidique, notamment pour qu'il ne soit pas injecté dans la région d'analyse. Le fluide évacué n'a pas de fonction particulière. Du fait des différentes architectures fluidiques, du liquide dit « purgé » peut ne pas encore avoir encore passé l'orifice de purge 219 (mais peut le passer par la suite, une fois l'injection terminé ou une fois une séquence de nettoyage enclenchée).

Les figures 6 et 7 illustrent respectivement schématiquement et plus réalistement un circuit fluidique 600 qui est structurellement similaire à celui des documents WO2023036805, WO2023036806, WO2023036808, WO2023036809.

Les figures 8 à 15 illustrent schématiquement et plus réalistement différents modes de réalisation et variantes de circuit fluidiques.

### Capteur de présence de fluide

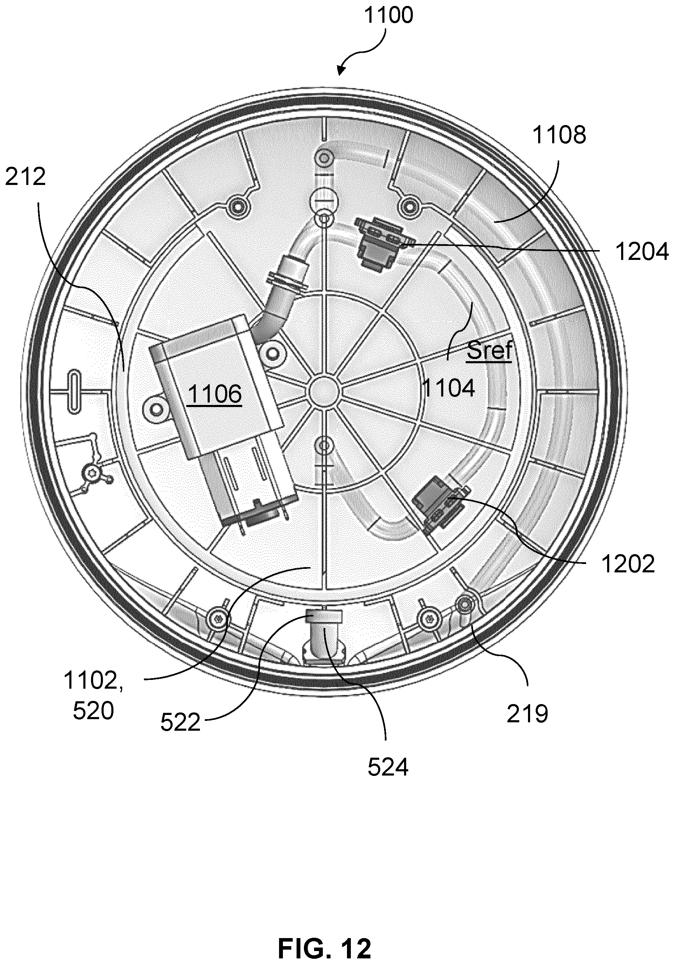

Comme illustré en exemple sur la figure 12 (mais non visible sur la figure 7), la station 200 peut comprendre au moins un capteur 1202, 1204 de présence de fluide le long du circuit fluidique. Dans un mode de réalisation, la station 200 comprend deux capteurs 1202, 1204 de présence de fluide espacés le long du circuit fluidique et définissant ainsi une section de référence Sref (dont le volume prédéterminé est connu par la circuiterie de contrôle 1600), ce qui permet de s'assurer qu'un volume minimum d'urine (le volume prédéterminé donc) a été prélevé. Par exemple, le volume prédéterminé est compris entre 40 et 50 microlitres (notamment aux environs de 40 microlitres). En mesurant le temps de déplacement du fluide entre les deux capteurs 1202, 1204 de présence de fluide, la circuiterie de contrôle 1600 peut calculer le débit de la pompe

Le ou les deux capteurs 1202, 1204 de présence de fluide peuvent comprendre des électrodes ou des sondes optiques. Le document WO2022184984 décrit en détail de tels capteurs (notamment le capteur optique).

Tout circuit fluidique décrit dans ce document peut comprendre au moins deux capteurs 1202, 1204 de présence de fluide.

### Circuit fluidiques, modes de réalisation et variantes

Plusieurs autres modes de réalisation et variantes du circuit fluide vont être décrits à présent.

### Définitions

Le terme « prélèvement » signifie l'introduction d'urine dans le circuit fluidique par l'extrémité de prélèvement, depuis le réservoir, via notamment l'orifice de prélèvement. Le volume total d'urine prélevé par miction peut être compris entre 100 et 500 microlitres, par exemple aux environs de 200 microlitres.

Le terme « injection » signifie l'introduction d'urine dans la région d'analyse par l'extrémité d'injection, c'est-à-dire notamment la mise en contact d'urine avec un réactif (étape d'injection). L'injection se fait durant une étape d'injection, lorsque l'extrémité d'injection est en position d'injection.

Le terme de « purge » signifie la mise à l'écart dans le circuit hydraulique, de sorte que le liquide ne peut plus être injecté dans la région d'analyse (étape de purge). La purge peut comprendre de l'urine qui reste dans le circuit fluidique temporairement, avant d'être évacuée plus tard. La purge se fait durant une étape de purge.

L'expression « premiers volumes d'urine prélevés » désignent les premiers volumes d'urine qui pénètrent dans le circuit fluidique par l'orifice de collecte 218. Typiquement, les premiers volumes d'urine prélevés sont compris entre 50 et 150 microlitres.

L'expression « derniers volumes d'urine prélevés » désignent les derniers volumes d'urine qui pénètrent dans le circuit fluidique par l'orifice de collecte 218. Typiquement, les derniers volumes d'urine prélevés sont compris entre 50 et 150 microlitres.

L'expression « volumes intermédiaires d'urine prélevés » désignent les volumes d'urine intermédiaires qui pénètrent dans le circuit fluidique par l'orifice de collecte 218, c'est-à-dire ni les premiers volumes prélevés, ni les derniers volumes prélevés ». Typiquement, les volumes intermédiaires d'urine prélevés sont compris entre 50 et 150 microlitres.

L'expression « volumes d'urine injectés » désignent les volumes d'urine qui sont mis dans la zone de réaction par l'extrémité d'injection, c'est-à-dire notamment en contact avec du réactif urinaire. Le volume d'urine injecté (en une ou plusieurs fois sur une même région d'analyse) peut être compris entre 10 et 50 microlitres.

Sur les figures 6 et 8 à 10, les flèches creuses symbolisent schématiquement la présence d'urine et le sens de circulation du fluide et les flèches en trait plein symbolisent un mouvement de l'extrémité d'injection ou de la cartouche.

Grâce aux capteurs 1202, 1204 de présence de fluide, la circuiterie de contrôle 1600 peut mesurer la valeur du débit de la pompe. De plus, la circuiterie de contrôle 1600 connait les différents volumes internes du circuit fluidique (distance et/ou section des composants du circuit fluidique), qui sont prédéterminés. Par exemple, le volume entre le capteur 1202 de présence de fluide le plus proche de l'extrémité de prélèvement et l'extrémité de prélèvement est compris entre 50 et 80 microlitres (par exemple environ 70 microlitres). Ainsi, la circuiterie de contrôle 1600 connait le débit d'urine dans le circuit fluidique, ainsi que la position de l'urine dans le circuit fluidique par rapport aux capteurs de présence de fluide (au niveau du premier capteur, entre les deux capteurs, au niveau du deuxième capteur, après le deuxième capteur ; les termes relatifs).

Les circuits hydrauliques décrits ici fonctionnent en microfluidique, où les forces de capillarités sont supérieures à celle de la gravité. Par conséquent, en l'absence de fonctionnement de la pompe, le liquide reste immobile dans le circuit fluidique. En outre, les volumes d'urine prélevés ne se mélangent pas spontanément lorsqu'ils se déplacement au sein du circuit fluidique. On considère que les déplacements de particule au sein de l'urine sont négligeables par rapport au déplacement de l'urine elle-même.

### Description générale des modes de réalisation

Dans un mode de réalisation dite « sacrificiel », le circuit fluidique est configuré pour sacrifier les premiers volumes d'urine prélevés dans le réservoir par l'extrémité de prélèvement. Autrement dit, le dispositif d'urine 100 n'injecte pas dans la région d'analyse les premiers volumes d'urine prélevés, qui sont purgés.

Dans un autre mode de réalisation dit « injecteur-préleveur », qui peut être complémentaire au mode de réalisation sacrificiel du circuit fluidique, l'extrémité d'injection est utilisée comme extrémité de prélèvement, par laquelle de l'urine pénètre dans le circuit fluidique.

Dans un autre mode de réalisation dit « double-sens », qui peut être complémentaire aux modes de réalisation sacrificiel et injecteur-préleveur, le circuit fluidique comprend une section de changement de sens de déplacement de l'urine, à l'intérieur de laquelle l'urine prélevée change de sens de circulation : un front avant d'un volume d'urine dans la section de changement de sens de déplacement de l'urine devient un front arrière de ce même volume d'urine (et inversement, le front arrière devient le front avant).

Différents exemples de circuits fluidiques vérifiant au moins l'un des modes de réalisation vont être présentés. Les figures 8 à 11 sont schématiques et doivent être contemplées à l'aune de l'architecture plus précise fournie en figure 6 ou en figure 7.

### Mode de réalisation sacrificiel

Un circuit fluidique 600 conforme au mode de réalisation sacrificiel (ce mode de réalisation n'est pas injecteur-préleveur ni double-sens) va être à présent décrit.

Le circuit fluidique 600 des figures 6 et 7 comprend, en série, l'orifice de collecte 218, une extrémité de prélèvement 602, reliée à de la tuyauterie 604 qui mène jusqu'à la pompe 606, puis de la tuyauterie 608 reliée à une extrémité d'injection 510. Le circuit fluidique 600 comprend outre l'extrémité de purge 610, dans lequel l'extrémité d'injection 510 peut déverser du fluide pour purger le circuit. L'extrémité de purge 610 est ainsi à l'opposé de l'extrémité de prélèvement 602, le long du circuit fluidique 600.

Dans le circuit fluidique 600, le fluide circule dans un seul et même sens, de l'orifice de collecte 218 à l'extrémité d'injection 510. En fonction de la position de l'extrémité d'injection 510 (qui est mobile en translation), le fluide du circuit fluidique 600 peut se déverser la région d'analyse 508 (en position d'injection avec une position appropriée de la cartouche 202) ou dans l'extrémité de purge 610 (en position de purge). La pompe 606 peut donc fonctionner dans un seul sens de circulation de fluide, depuis l'extrémité de prélèvement jusqu'à l'extrémité de purge, en passant par l'extrémité d'injection. On parle de direction de déplacement unidirectionnelle, de l'extrémité de prélèvement 602 vers l'extrémité d'injection 510.

Un septum 512 peut être prévu pour étanchéifier l'orifice de collecte 218 de l'humidité ambiante. Le septum 512 peut être traversé par l'extrémité d'injection 510 en position de collecte d'urine.

Le circuit fluidique 600 est linéaire, en ce sens qu'il n'y a pas de bifurcation ou de jonction fluidique ce qui le rend particulièrement simple, étanche, et facile d'utilisation.

La gestion de la pompe 606 et de la position de l'extrémité d'injection 610 est faite par la circuiterie de contrôle 1600.

Dans ce circuit fluidique 600, la région d'analyse 508 et l'extrémité de purge 610 sont arrangées à proximité pour que l'extrémité d'injection 510 puis sélectivement y déverser du fluide. En particulier, lorsque l'extrémité d'injection 510 est mobile en translation, l'extrémité d'injection 510, la région d'analyse 508 et l'extrémité de purge 610 sont alignées selon la direction de translation.

Comme illustré en figure 6 a), lors d'une étape de prélèvement, la pompe 606 est activée et l'urine pénètre par l'orifice de captation 218 dans l'extrémité de prélèvement 602, puis via la tuyauterie 602, 606, jusqu'à l'extrémité d'injection 510 (figure 6 a). L'extrémité d'injection peut être en position d'attente ou en position de purge.

Les premiers volumes prélevés parcourent en premier le circuit fluidique 600. A ce titre, ils peuvent être contaminés par des résidus d'urine d'un prélèvement précédent. Il est donc préférable de ne pas injecter ces premiers volumes d'urine sur le réactif urinaire.

A cette fin, comme illustré en figure 6 b), lors d'une étape de sacrifice, la circuiterie de contrôle 1600 met ou maintient en position de purge l'extrémité d'injection 510, de sorte que l'extrémité d'injection 510 communique avec l'extrémité de purge 610. La circuiterie de contrôle 1600 actionne la pompe pour évacuer les premiers volumes d'urine prélevés vers l'extrémité de purge 610.

Les capteurs 1202, 1204 de présence de fluide, qui permettent de connaitre le débit de la pompe, permettent de connaitre le volume évacué (la circuiterie de contrôle 1600 peut stocker la valeur du volume du circuit fluidique entre un capteur 1202, 1204 de présence de fluide et l'extrémité d'injection).

Ensuite, comme illustré en figure 6 c), lors d'une étape de transition, la circuiterie de contrôle 1600 met l'extrémité d'injection 510 en position neutre et positionne la cartouche pour que la région d'analyse 508 désirée soit en position pour recevoir de l'urine par l'extrémité d'injection 510.

Enfin, comme illustré en figure 6 d), lors d'une étape d'injection, la circuiterie de contrôle 1600 réactive la pompe 606 et l'extrémité d'injection 510 injecte des volumes d'urine prélevé, qui correspondent à des volumes intermédiaires d'urine prélevés , dans la région d'analyse 508. Ainsi, les premiers volumes prélevés, et potentiellement contaminés, sont sacrifiés dans l'extrémité de purge et ne sont pas utilisés pour l'analyse d'urine.

Enfin, comme illustré en figure 6 e), lors d'une étape de purge, la circuiterie de contrôle 1600 positionne l'extrémité d'injection en position de purge pour permettre la purge du circuit fluidique 1600. L'extrémité d'injection 510 déverse ainsi le fluide dans l'extrémité de purge 610. Dans une étape, préalable, de transition, la circuiterie de contrôle 1600 peut mettre l'extrémité d'injection 520 en position neutre pour l'étape de purge. Dans cette étape de purge, les premiers volumes prélevés sont déjà dans l'extrémité de purge (ou déjà purgées via l'orifice de purge).

Le circuit fluidique 600 est particulièrement simple à réaliser.

### Mode de réalisation injecteur-préleveur

Le circuit fluidique 800 est conforme au mode de réalisation injecteur-préleveur.

Dans ce mode de réalisation, l'extrémité d'injection est utilisée comme extrémité de prélèvement, c'est-à-dire que de l'urine parcourt l'extrémité de prélèvement dans un sens pour le prélèvement puis parcourt l'extrémité de prélèvement pour l'injection et les premiers volumes d'urine prélevés restent sur le premier front. Il n'y a donc pas de section de changement de sens de déplacement de l'urine à l'intérieur de laquelle l'urine prélevée change de sens de déplacement (avec inversion des fronts avant et arrière, comme décrit précédemment).

Le circuit fluidique 800 comprend en série l'orifice de collecte 218, l'extrémité de prélèvement 802 (qui est l'extrémité d'injection 520) que de la tuyauterie 804 relie à une valve 806, qui est elle-même reliée à de la tuyauterie 808 reliée à une pompe 810, qui est reliée à de la tuyauterie 812 reliée à la valve 806, reliée à une extrémité de purge 814 connecte à l'orifice de purge 219.

La valve 806 est configuré pour inverser les connexions entre d'un côté les tuyauteries 804, 808 et de l'autre côté de la tuyauterie 812 et de l'extrémité de purge 814.

La valve 806 peut être une valve dite « 4 voies 2 positions », avec 2 paires de ports qui sont sélectivement reliés les uns aux autres. La valve 806 peut comprendre un ensemble d'élément hydrauliques fluidiquement et fonctionnellement équivalents.

Le circuit fluidique 800 forme une boucle entre l'extrémité de prélèvement 802 et l'extrémité d'injection 520.

Dans le circuit fluidique 800, l'extrémité de prélèvement 802 est confondue avec l'extrémité d'injection 520, en ce sens où le prélèvement de fluide depuis l'orifice de collecte 218 est effectué par l'extrémité d'injection 520. Dit autrement, l'extrémité d'injection 520, configurée pour injecter de l'urine dans la région d'analyse 508, sert d'extrémité de prélèvement 802.

Dans ce circuit fluidique 800, la région d'analyse 508 et la réservoir 524 sont arrangés à proximité pour que l'extrémité d'injection 510 (qui est aussi l'extrémité de prélèvement 802) puisse sélectivement y déverser de l'urine ou y prélever de l'urine. En particulier, lorsque l'extrémité d'injection 520 est mobile en translation, l'extrémité d'injection 520, la région d'analyse 508 et le réservoir 524 sont alignés selon la direction de translation.

Comme le circuit fluidique est par moment déconnecté de l'orifice de collecte 218, ce dernier est étanchéifié de l'intérieur du boitier par le septum 524 (visible en figure 5 B), que l'extrémité d'injection 520 (ici l'extrémité de prélèvement 802 donc) peut traverser en position de collecte d'urine. A cette fin, l'extrémité d'injection peut comprendre une aiguille, et notamment une aiguille biseautée.

Les capteurs 1202, 1204 de présence d'urine sont typiquement disposés au niveau de la tuyauterie 808 (au-delà de la valve 806 donc).

Comme illustré sur la figure 8 a), lors d'une étape de prélèvement, la circuiterie de contrôle 1600 met l'extrémité d'injection 520 en position de prélèvement et la pompe 808 aspire de sorte que l'urine pénètre dans le circuit fluidique 800. La tuyauterie 804, 808, 812 est suffisamment longue pour stocker suffisamment d'urine prélevée (avec notamment la portion entre les deux capteurs 1202, 1204 de présence de fluide).

Ensuite, comme illustré sur la figure 8 b), lors d'une étape de transition, la circuiterie de contrôle 1600 met l'extrémité d'injection 520 en position d'injection, une fois que la région d'analyse 508 est disposé devant l'extrémité d'injection 520. La circuiterie de contrôle 1600 pilote aussi la valve 806 pour inverser les positions et relier la tuyauterie 812 à la tuyauterie 804 via la valve 806.

Sur la figure 8 c), lors d'une étape d'injection, la circuiterie de contrôle 1600 active à nouveau la pompe 810 et l'urine continue d'avancer dans le même sens. Du fait de l'inversion de la valve 806, l'urine prélevée est redirigée vers l'extrémité d'injection 520 qui injecte ensuite sur la région d'analyse 508.

Enfin, comme illustré en position 8 d), lors d'une étape de purge, la circuiterie de contrôle 1600 pilote la valve 806 pour ré-inverser les positions et ainsi permettre de purger l'urine restante dans l'extrémité d'injection 520 et la tuyauterie 804, 808, 812 vers l'orifice de purge 219. La circuiterie de contrôle 1600 peut aussi mettre l'extrémité d'injection 510 en position neutre (notamment pour laisser de l'air entrer dans le circuit).

Le circuit hydraulique 800 est compact puisque l'extrémité d'injection 520 et l'extrémité de prélèvement 802 sont une seule et même pièce. En outre, l'extrémité d'injection 520 est nettoyée lors du prélèvement par les premiers volumes prélevés.

Dans cette variante, les premiers volumes prélevés sont les premiers volumes injectés.

### Mode de réalisation injecteur-préleveur et sacrificiel

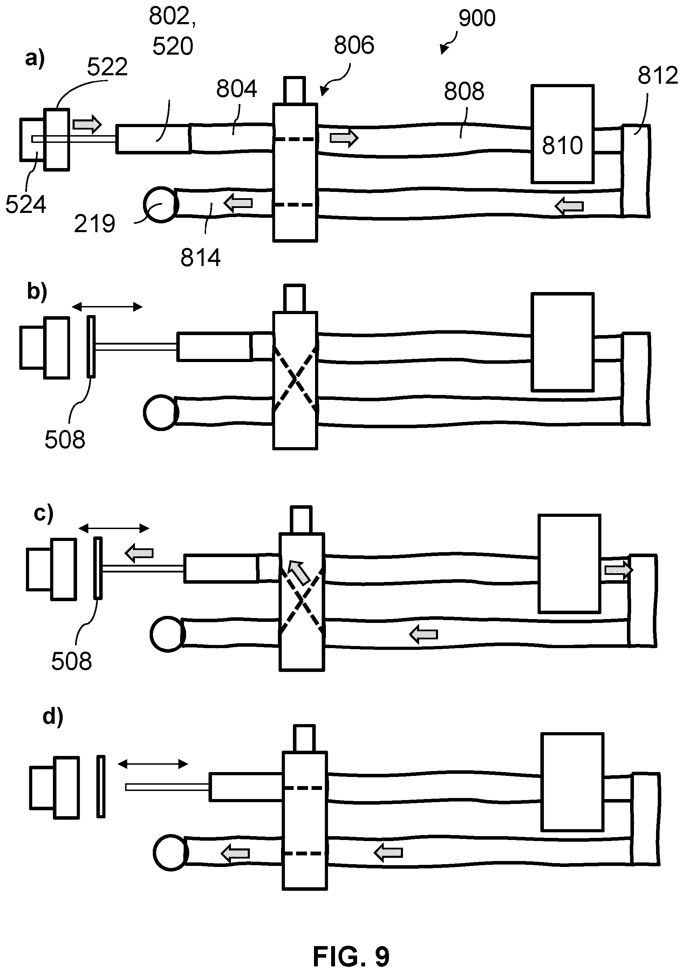

La figure 9 illustre une variante 900 du circuit fluidique 800 qui est en plus conforme au mode de réalisation sacrificiel. Les références numériques sont identiques à celle du circuit fluidique 800 mais la logique d'activation de la valve et/ou de la pompe change.

Pour encore limiter la cross-contamination et ne pas injecter les premiers volumes prélevés, dans une étape de sacrifice, la circuiterie de contrôle 1600 peut activer la position de la valve 806 de la figure 9 b) uniquement une fois que les premiers volumes d'urines prélevés ont passé la valve 806 pour rejoindre l'extrémité de purge 814, comme représenté par la flèche dans l'extrémité de purge de la figure 9 a). De la sorte, les premiers volumes d'urine prélevés sont sacrifiés et les volumes d'urine injectés sont les volumes intermédiaires d'urine prélevés.

Pendant l'étape d'injection (figure 9 c), les premiers volumes d'urine sont donc dans l'extrémité de purge 814 et peuvent recirculés ensuite dans la tuyauterie 808, via la valve 806, pour *in fine* être purgés, comme décrit en position de la figure 9 d). Ainsi, dans l'étape de purge (figure 9 d), les premiers volumes prélevés sont déjà dans l'extrémité de purge (ou déjà purgées via l'orifice de purge).

### Mode de réalisation double-sens

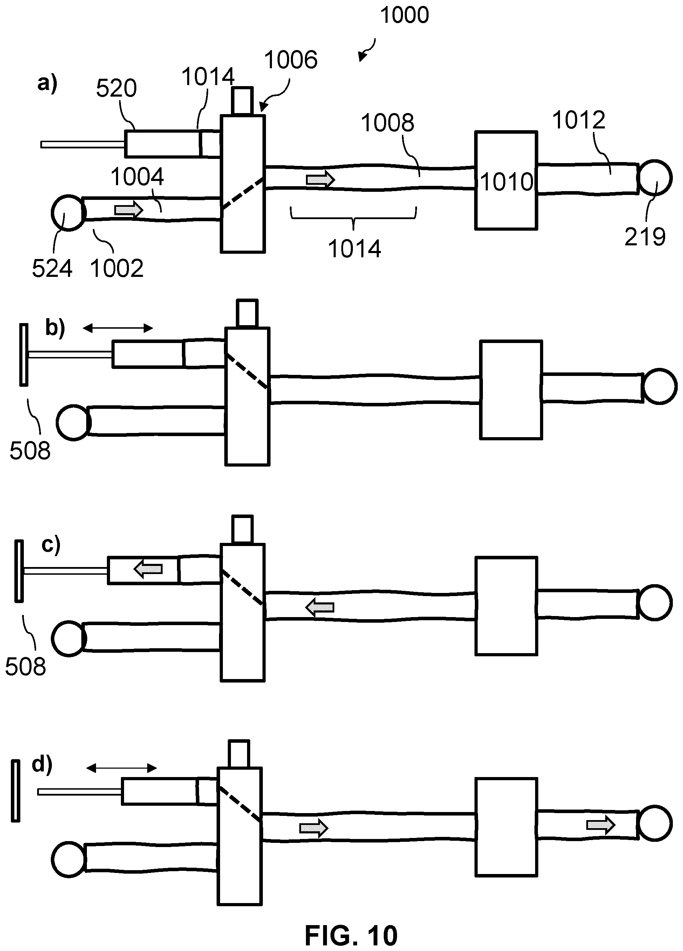

Le circuit fluidique 1000 de la figure 10 est conforme au mode de réalisation double-sens, ainsi que, facultativement, conforme au mode de réalisation sacrificiel (notamment en ce qu'il est structurellement conçu pour une mise à en oeuvre naturelle du mode de réalisation sacrificiel).

Dans ce mode de réalisation, le circuit fluidique 1000 comprend une section de changement de sens de déplacement de l'urine, à l'intérieur de laquelle l'urine prélevée modifie son sens de circulation, c'est-à-dire qu'à l'intérieur de la section de changement de sens de déplacement de l'urine un front avant d'un volume d'urine devient le front arrière de ce volume d'urine. L'intérêt d'un tel changement de sens sera expliqué par la suite.

Le circuit fluidique 1000 comprend en série l'orifice de collecte 218 relié à l'extrémité de prélèvement 1002 que de la tuyauterie 1004 relie à une valve 1006, qui est elle-même reliée à de la tuyauterie 1008 reliée à une pompe 1010, qui est reliée à une extrémité de purge 1012. En outre, en parallèle, la valve 1006 est reliée aussi à l'extrémité d'injection 520 via de la tuyauterie 1014.

La valve 1006 est configurée pour sélectivement relier la tuyauterie 1008 à la tuyauterie 1004 (vers l'extrémité de prélèvement 1002 donc) ou la tuyauterie 1014 (vers l'extrémité d'injection 520 donc).

La valve 1006 peut être une valve dite « 2 voies 2 positions », avec deux entrées sélectivement reliées à une sortie.

Dans ce mode de réalisation, la pompe 1010 est configurée pour faire déplacer le fluide dans le circuit fluidique 1000 sélectivement dans un sens ou dans l'autre.

Les capteurs 1202, 1204 de présence de fluide sont typiquement disposés au niveau de la tuyauterie 1008 (au-delà de la valve 1006 donc).

Comme illustré en figure 10 a), dans une étape de prélèvement, la circuiterie de contrôle 1600 met (ou maintient) la valve 1006 en position reliant l'extrémité de prélèvement 1002 à la tuyauterie 1008 et active la pompe 1010, de sorte que l'extrémité de prélèvement 1002 prélève du liquide depuis le réservoir 524. Les volumes prélevés pénètrent dans la tuyauterie 1008.

Une fois une quantité suffisante prélevée, comme illustré en figure 10 b), dans une étape de transition, la circuiterie de contrôle 1600 met l'extrémité d'injection 520 en position d'injection, une fois que la région d'analyse 508 est disposée devant l'extrémité d'injection 520.

Ensuite, comme illustré en figure 10 c), dans une étape d'injection, la circuiterie de contrôle 1600 inverse le sens de fonctionnement de la pompe 1010, de sorte que l'urine change de sens au sein de la tuyauterie 1008, pour retraverser la valve 1006 et rejoindre la tuyauterie 1014 et l'extrémité d'injection 520.

Le changement de sens signifie que les premiers volumes d'urine prélevés, qui étaient à l'avant du front d'urine dans le circuit fluidique, se retrouve être à l'arrière, et les derniers volumes d'urine prélevés, qui étaient à l'arrière du front d'urine dans le circuit fluidique, se retrouve à l'avant.

Ainsi, les derniers volumes prélevés ou les volumes intermédiaires d'urine prélevés, qui sont moins susceptibles à la cross contamination, se retrouvent être les premiers volumes injectés dans la région d'analyse 508. On peut ainsi parler ainsi de logique LIFO ("last in - first out" en anglais, ou dernier entré - premier sorti en français) dans le premier cas.

Sur la figure 10 d), dans une étape de purge, la circuiterie de contrôle 1600 change à nouveau le sens de fonctionnement de la pompe 1010 de sorte que l'urine encore présente dans le circuit fluidique 1000 est purgée vers l'orifice de purge 219. La circuiterie de contrôle 1600 peut aussi rechanger la position de la valve 1006 pour purger tout le circuit. Dans une étape, préalable, de transition, la circuiterie de contrôle 1600 peut mettre l'extrémité d'injection 520 en position neutre pour l'étape de purge.

Dans cette étape de purge, les premiers volumes prélevés passent dans l'extrémité de purge.

Dans une variante, la position de la figure 10 c) est mise en oeuvre alors que de l'urine prélevée est encore dans l'extrémité de prélèvement 1002 et/ou la tuyauterie 1004 (ce sont les derniers volumes prélevés). Dans ce cas, les volumes d'urine injectés correspondent à des volumes intermédiaires d'urine prélevés. Cette urine présente une qualité similaire aux derniers volumes d'urine prélevés.

Le circuit fluidique 1000 comprend ainsi une section de changement de sens de déplacement de l'urine 1014, à l'intérieur de laquelle les fronts d'un volume d'urine s'inverse : le front avant devient le front arrière et inversement. La section de changement de sens de déplacement de l'urine est ici une portion du la tuyauterie 1008.

Le circuit fluidique 1000 peut ainsi présenter une section linéaire (sans jonction donc) de changement de sens, au sein de laquelle le fluide inverse son sens de circulation : le fluide circule donc dans l'autre sens (cela est à distinguer d'une situation où une boucle permet au fluide de circuler en sens contraire mais le fluide ne change pas de sens dans la portion, la boucle n'étant pas une portion linéaire puisqu'il faut une jonction - voir par exemple la tuyauterie 804 de la figure 8, qui est parcouru par de l'urine dans les deux sens mais l'urine effectue une boucle par la valve 806 et la tuyauterie 808, 812). Par sens de circulation du fluide, il est entendu le sens dans lequel le débit est positif, c'est-à-dire le sens dans lequel les éléments constituant le fluide circulent en majorité.

Dans le mode de réalisation de la figure 10, l'extrémité de prélèvement 1002 et l'extrémité d'injection 520 sont deux pièces distinctes.

Lorsque deux capteurs 1202, 1204 de présence de fluide sont disposés le long du circuit fluidique, la section de changement de déplacement d'urine peut comprendre la portion du circuit fluidique entre les deux capteurs 1202, 1204 de présence de fluide. En effet, l'inversion de sens peut être effectué une fois que les deux capteurs 1202, 1204 de présence de fluide ont identifié de l'urine, de sorte que la circuiterie de contrôle sait qu'un volume suffisant d'urine a été prélevé.

### Mode de réalisation double-sens et sacrificiel

Le circuit fluidique 1000 de la figure 10 peut aussi être conforme au mode de réalisation sacrificiel.

Il suffit à cet égard que l'étape d'injection de la figure 10 c) s'arrête avant que les premiers volumes d'urine prélevés ne soient injectés dans la région d'analyse 508. La purge se fait ensuite conformément à la figure 10 c).

### Mode de réalisation injecteur-préleveur, double-sens et typiquement sacrifiel

Un circuit fluidique 1100 conforme aux modes de réalisation injecteur-préleveur, double-sens et, facultativement, sacrificiel (notamment en ce qu'il est structurellement conçu pour une mise à en oeuvre naturelle du mode de réalisation sacrificiel) va être décrit.

Ce circuit fluidique 1100 présente de nombreux avantages : la cross-contamination est minimisée puisque les volumes d'urine injectés ne passent que dans des portions du circuit fluidique qui ont été rincées par les premiers volumes prélevés.

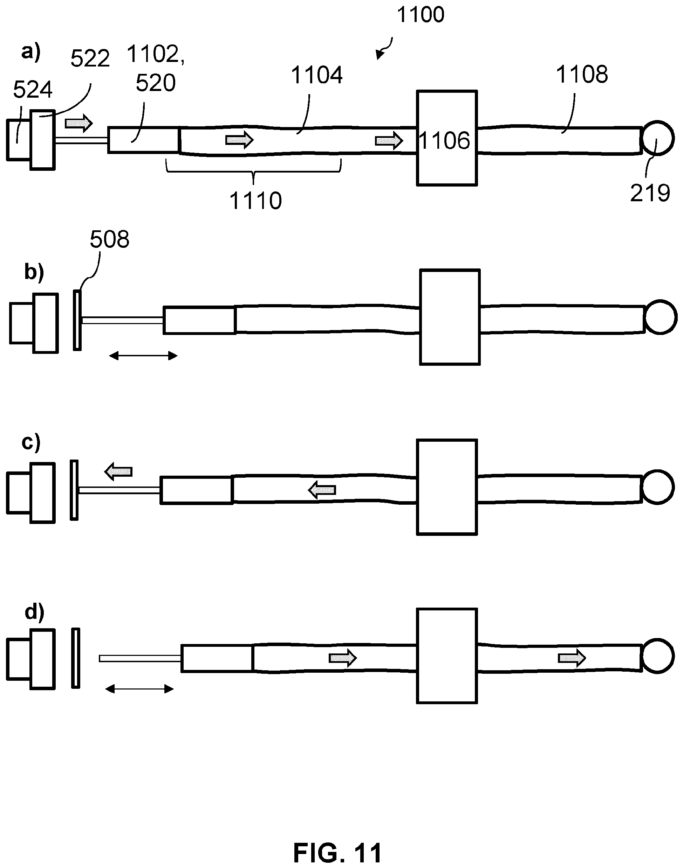

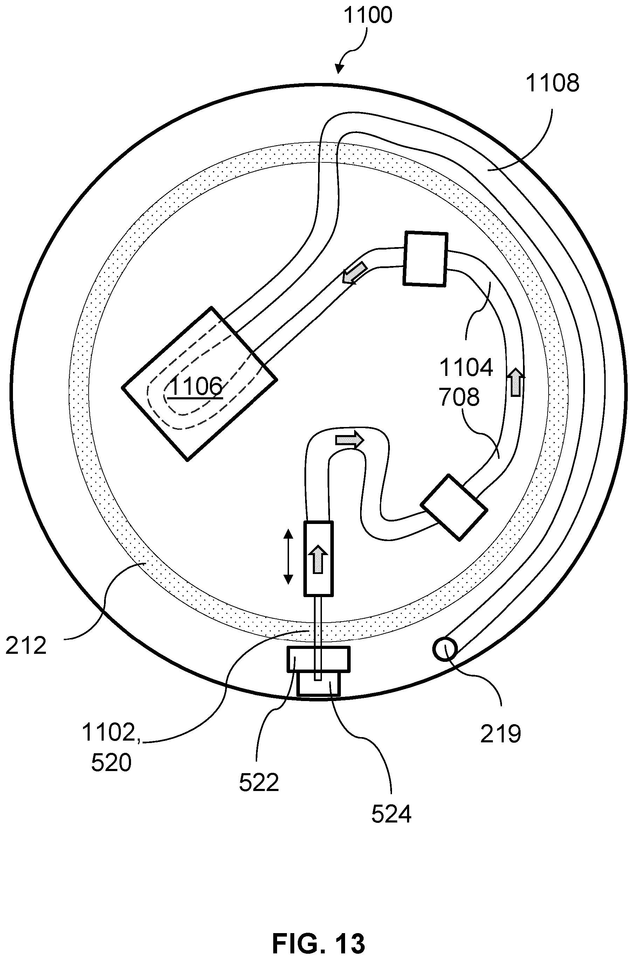

La figure 11 illustre de façon schématique le circuit fluidique 1100 ; la figure 12 illustre une version d'implémentation dans le dispositif 100 du circuit fluidique 1100 (avec les positionnements relatifs des composants) et les figures 13 à 15 représentent une version simplifiée de la figure 12 avec les étapes de la figure 11.

Le circuit fluidique 1100 comprend en série l'orifice de collecte 218 (avec ici le septum 522 et le réservoir 524 décrit en relation avec la figure 8), relié à l'extrémité de prélèvement 1102 qui est aussi l'extrémité d'injection 520, elle-même reliée à de la tuyauterie 1104 qui mène jusqu'à la pompe 1106, puis une extrémité de purge 1108 qui relie à l'orifice de purge 219.

La pompe 1106 est configurée pour faire déplacer le fluide dans le circuit fluidique 1100 sélectivement dans un sens ou dans l'autre

Le circuit fluidique 1100 est linéaire, en ce sens qu'il n'y a pas de bifurcation ou de jonction fluidique ce qui le rend particulièrement simple, étanche, et facile d'utilisation.

Dans ce circuit fluidique 1100, la région d'analyse 508 et la réservoir 524 sont arrangés à proximité pour que l'extrémité d'injection 520 (qui est aussi l'extrémité de prélèvement 802) puisse sélectivement y déverser de l'urine ou y prélever de l'urine. En particulier, lorsque l'extrémité d'injection 520 est mobile en translation, l'extrémité d'injection 520, la région d'analyse 508 et le réservoir 524 sont alignés selon la direction de translation.

Les deux capteurs 1202, 1204 de présence de fluide sont typiquement disposés au niveau de la tuyauterie 1104 ou 1108.

Comme illustré en figures 11 a) et 13, dans une étape de prélèvement, la circuiterie de contrôle 1600 met ou maintient l'extrémité d'injection 520 en position de prélèvement et active la pompe 1106 de sorte que l'urine est prélevée depuis l'orifice de collecte 218 vers l'extrémité d'injection 520 puis la tuyauterie 1104. Les volumes d'urine prélevés sont ainsi dans la tuyauterie 1104. Typiquement, le prélèvement se fait jusqu'à ce que le capteur de présence de fluide 1202, 1204 détecte de l'urine. Ainsi, il est assuré que le volume constitué par la tuyauterie entre les deux capteurs d'urine est empli d'urine.

Ensuite, comme illustré sur la figure 11 b), dans une étape de transition, la circuiterie de contrôle 1600 met l'extrémité d'injection 520 en position d'injection, une fois que la région d'analyse 508 est disposé devant l'extrémité d'injection 520.

Ensuite, comme illustré en figure 11 c) et 14, dans une étape d'injection, la circuiterie de contrôle 1600 active la pompe 1106 de sorte que l'urine prélevée change de sens de circulation au sein de la tuyauterie 1104. On a ainsi une section 1110 de changement de sens de déplacement de l'urine, qui comprend au moins une portion de la tuyauterie 1104 (et qui peut même comprendre l'extrémité d'injection 520). En l'occurrence, la section 1110 comprend notamment la portion du circuit fluidique (ici de la tuyauterie 1104) comprise entre les deux capteurs de présence de fluide. L'extrémité d'injection 520 est donc parcourue par de l'urine dans un sens, lors du prélèvement (les premiers volumes prélevés étant sur le front avant) et dans l'autre sens, lors de l'injection (les derniers volumes d'urine prélevés étant sur le front avant).

L'injection est typiquement contrôlée de sorte que toute l'urine prélevée ne soit pas injectée dans la région d'analyse 508. En particulier, la circuiterie de contrôle 1600 arrête la pompe 606 avant que les premiers volumes prélevés ne soient injectés. Dans ce cas, le circuit fluidique est conforme au mode de réalisation sacrificiel.

Enfin, comme illustré en figure 11 d) et 15, dans une étape de purge, la circuiterie de contrôle 1600 change le sens de fonctionnement de la pompe 606 pour purger le circuit fluidique 1100 en dirigeant l'urine restante vers l'orifice de purge 219. Dans une étape, préalable, de transition, la circuiterie de contrôle 1600 peut mettre l'extrémité d'injection 520 en position neutre pour l'étape de purge.

Grâce à ce changement de sens, les derniers volumes d'urine prélevés ou les volumes intermédiaires prélevés (qui sont les plus propres car les premiers volumes d'urine prélevés ont nettoyé le circuit fluidique 1100) deviennent les volumes d'urine injectés. De plus, grâce au fait que l'extrémité d'injection 520 est l'extrémité de prélèvement 1102 et grâce au circuit fluidique 1100 à double-sens, le chemin parcouru dans le circuit fluidique 1100 par les volumes d'urine injectés a été nettoyés par les premiers volumes d'urine prélevés.

Enfin, en arrêtant l'injection avant que les premiers volumes d'urine prélevés ne soient injectés, il est assuré que les premiers volumes d'urine prélevés sont sacrifiés.

Ce circuit fluidique 1100 présente de nombreux avantages qui seront détaillés par la suite. En particulier, ce circuit fluidique 1100 permet de significativement diminuer les risques de cross-contamination pour trois raisons : le circuit fluidique qui intervient pour injecter a été nettoyé pendant le prélèvement (puisque l'extrémité d'injection sert d'extrémité de prélèvement). Enfin ce circuit fluidique 1100 permet d'injecter dans la région d'analyse 508 de l'urine dernièrement prélevée, donc de l'urine qui parcourt un circuit fluidique déjà entièrement nettoyé.

Les premiers volumes d'urine injectée correspondent aux derniers volumes d'urine prélevés ou aux volumes intermédiaires d'urine prélevés et la portion du circuit fluidique parcouru par les volumes injectés a été entièrement parcouru par les premiers volumes d'urine prélevée (notamment l'extrémité d'injection 520). Le détail de ce circuit sera expliqué par la suite, avec notamment des variantes.

Dans les modes de réalisation décrit, la région d'analyse 508 peut être mis en position par rotation de la cartouche 202 dans le logement 212.

### Précharge

Pour les modes de réalisation décrit précédemment, à l'exception de celui des figures 6 et 7, une variante avec précharge peut être mise en oeuvre. Lors du prélèvement, un mélange d'air et d'urine peut être prélevé, notamment dû à l'irrégularité d'arrivée d'urine dans le réservoir. Par conséquent, le circuit fluidique peut alterner entre urine et air. Pour l'injection, il est préférable de n'expulser que de l'urine.

Grâce aux deux capteurs 1202, 1204 de présence de fluide, l'étape de prélèvement peut s'arrêter quand la section de référence Sref entre les deux capteurs 1202, 1204 de présence de fluide est empli d'urine (sans présence de front d'air). La précharge consiste à purger de l'urine par l'extrémité de prélèvement 510, 520, pour que l'urine présente dans la section de référence Sref soit amenée au niveau de l'extrémité de prélèvement. La précharge peut ainsi comprendre une évacuation de 80 microlitres d'urine. Ce volume est déterminé en fonction des volumes précités dans la description. Les volumes injectés sont donc des volumes intermédiaires d'urine prélevés.

Dans le mode de réalisation de la figure 8 ou 9, l'étape de précharge se fait avant l'étape de transition de la figure 8 b) ou 9 b). Dans ce cas, la circuiterie de contrôle 1600 laisse l'extrémité d'injection 520 en position de prélèvement mais commute la valve 806 pour inverser les positions par rapport aux positions durant l'étape de prélèvement. La pompe 810 est ensuite réactivée pour vider un volume d'urine tel que décrit précédemment. Ainsi, les premiers volumes prélevés (pour le mode de réalisation de la figure 8) ou des volumes intermédiaires d'urine prélevés (pour le mode de réalisation de la figure 9) sont déchargés dans le réservoir 524. Ensuite, l'étape de transition de la figure 8 b) est mise en oeuvre (sauf que la valve 806 est déjà en place).

Dans le mode de réalisation de la figure 10, pour lequel une extrémité de purge telle que celle des figures 8, 9 et 11 est en outre prévue, l'étape de précharge se fait avant l'étape de transition de la figure 10 b). Dans ce cas, la circuiterie de contrôle 1600 met l'extrémité d'injection 520 dans une position de purge (non visible sur la figure 10, l'extrémité de purge n'est pas accessible directement par l'extrémité d'injection) et commute la valve 1006 pour inverser les positions par rapport aux positions durant l'étape de prélèvement. La pompe 1010 est ensuite activée en sens inverse pour vider un volume d'urine tel que décrit précédemment dans l'extrémité de purge. Ainsi, les dernières volumes prélevées (pour le mode de réalisation de la figure 9) ou des volumes intermédiaires d'urine prélevés (pour la variante non illustrée où de l'urine se trouve dans la tuyauterie 1004) sont déchargés dans le réservoir 524. Ensuite, l'étape de transition de la figure 10 b) est mise en oeuvre (sauf que la valve 1006 est déjà en place).

Dans le mode de réalisation de la figure 11, l'étape de précharge se fait avant l'étape de transition de la figure 11 b). Dans ce cas, la circuiterie de contrôle 1600 laisse l'extrémité d'injection 520 en position de prélèvement. La pompe 1106 est ensuite activée en sens inverse pour vider un volume d'urine tel que décrit précédemment dans le réservoir 524. Ainsi, les derniers volumes prélevés sont déchargés dans le réservoir 524. Ensuite, l'étape de transition de la figure 10 b) est mise en œuvre (sauf que la valve 1006 est déjà en place).

### Architecture et circuiterie de contrôle

La figure 16 illustre schématiquement une station 200 avec une circuiterie de contrôle 1600. La circuiterie de contrôle 1600 comprend un processeur 1602, une mémoire 1604 (RAM ou ROM, par exemple permanente) et une interface I/O (« in/out ») 1606 pour échanger des données.

La mémoire 1604 peut stocker des programmes exécutables par le processeur 1602.

La station 200 comprend en outre une batterie 1608 configurée pour alimenter en énergie les composants électriques ou électroniques de la station 200.

La circuiterie de contrôle 1600 peut notamment commander la pompe (référencée 606, 810, 1010, 1106), le moteur 702 pour déplacer la cartouche 202 dans la station 200, le moteur 704 pour déplacer l'extrémité d'injection 510, 520. La circuiterie de contrôle 1600 peut notamment échanger des informations avec le ou les capteurs 1202, 1204 de présence de fluide.

La station 200 peut en outre comprendre un module 1612 de communication sans-fil 1612 (par exemple un module *BlueTooth* ou *BlueTooth Low Energy*)*,* relié à la circuiterie de contrôle 1600. Le module 1612 permet d'échanger des informations (émission et réception), via un réseau de communication 1614 avec un terminal mobile 1616 (par exemple un smartphone) et/ou un serveur distant 1618. Le réseau de communication 1614 peut être sans-fil et/ou filaire et/ou un mélange des deux. Les données d'urine peuvent ainsi être envoyées au serveur 1618 et ensuite transmise au terminal mobile 1616 (ou bien communiquées directement au terminal mobile 1616 qui les transmet ensuite au serveur). Inversement, la station 200 peut recevoir des mises à jour depuis le serveur distant 1618 ou le terminal mobile 1616.

### Généralisation

Le circuit fluidique décrit dans la présente description s'applique de la même façon dans un dispositif dont la cartouche ne se déplace pas en rotation mais par exemple en translation.

L'extrémité d'injection, décrite comme étant mobile en translation, peut être mobile d'une autre façon, par exemple en en rotation.

Comme cela a été décrit, l'analyse d'urine peut se faire via un réactif ou sur l'urine directement.

## Revendications

1. Station (200) d'analyse d'urine comprenant :
- un boitier (204), configuré pour être placé entièrement dans une cuvette (106) de toilettes (102) et pour recevoir un jet d'urine, le boitier étant configuré pour recevoir à l'intérieur au moins une région d'analyse apte à recevoir de l'urine pour analyse,
- un circuit fluidique (1000, 1100), à l'intérieur du boitier (204), pour la circulation d'urine dans la station vers une région d'analyse (508),
- un analyseur (230), monté à l'intérieur du boitier (204), et configuré pour obtenir une information relative à l'urine dans la région d'analyse (508),
dans laquelle le circuit fluidique comprend une section (1110) de changement de sens de déplacement de l'urine, à l'intérieur de laquelle de l'urine change de sens de circulation, à l'intérieur de laquelle un front avant d'un volume d'urine devient un front arrière de ce volume d'urine.

2. Station selon la revendication 1, comprenant une pompe configurée pour faire changer le sens du fluide dans la section de changement de sens de déplacement de l'urine, par exemple par inversion le sens de fonctionnement de la pompe.

3. Station selon la revendication 1 ou 2, comprenant en outre un réservoir d'urine (524), configuré pour recevoir de l'urine issue d'une miction d'un utilisateur,
dans laquelle le circuit fluidique est pour la circulation d'urine dans la station depuis le réservoir d'urine (524) vers une région d'analyse (508),
dans lequel le circuit fluidique comprend une extrémité d'injection (520) configurée pour injecter de l'urine dans une région d'analyse (508) et une extrémité de prélèvement (802) configurée pour prélever de l'urine depuis le réservoir (524) et la faire pénétrer dans le circuit fluidique.

4. Station selon la revendication 3, dans lequel la logique du circuit fluidique est en « dernier entré - premier sorti », LIFO, de sorte que les derniers volumes d'urine prélevés par l'extrémité de prélèvement dans le réservoir (524) sont les premiers volumes d'urine injectés dans la région d'analyse par l'extrémité d'injection.

5. Station selon l'une quelconque des revendications 3 à 4, dans laquelle l'extrémité d'injection (520) est utilisée comme extrémité de prélèvement (802).

6. Station selon la revendication 5, dans laquelle la section de changement de sens de direction de l'urine comprend l'extrémité d'injection.

7. Station selon l'une quelconque des revendications précédentes, comprenant deux capteurs de présence de fluide (1112, 1114) disposé à deux endroits le long du circuit fluidique, dans lequel la section de changement de déplacement d'urine comprend la portion du circuit fluidique entre les deux capteurs de présence de fluide (1112, 1114).

8. Station selon l'une quelconque des revendications précédentes en combinaison avec la revendication 3, configurée pour sacrifier les premiers volumes d'urine prélevés dans le réservoir par l'extrémité de prélèvement, de sorte que la station n'injecte pas dans la région d'analyse (508) les premiers volumes d'urine prélevés.

9. Station selon l'une quelconque des revendications précédentes en combinaison avec la revendication 3, dans laquelle le réservoir et l'au moins une région d'analyse sont agencés à proximité dans la station.

10. Station selon l'une quelconque des revendications précédentes, comprenant un logement positionné à l'intérieur du boitier, configuré pour recevoir au moins partiellement une cartouche (202) comprenant l'au moins une région d'analyse.

11. Dispositif comprenant une station selon l'une quelconque des revendications précédentes, et une cartouche amovible de la station, dans lequel l'au moins une région d'analyse est montée sur la cartouche.

12. Méthode d'analyse d'urine à l'aide d'une station selon l'une quelconque des revendications 1 à 10 ou d'un dispositif selon la revendication 11, en combinaison avec la revendication 3, comprenant :
- une étape de prélèvement d'urine depuis le réservoir par l'extrémité de prélèvement,
- une étape d'injection d'urine dans la région d'analyse par l'extrémité d'injection,
dans laquelle entre l'étape de prélèvement et l'étape d'injection, l'urine change de direction d'écoulement dans la section de changement de sens de déplacement de l'urine.

13. Méthode selon la revendication 12, dans laquelle la station comprend une pompe, dans laquelle le sens de fonctionnement de pompe est inversée entre l'étape de prélèvement et l'étape d'injection.

14. Méthode selon la revendication 12 ou 13, comprenant entre l'étape de prélèvement et l'étape d'injection, une étape de précharge, au cours de laquelle de l'urine prélevée est renvoyée dans le réservoir par l'extrémité de prélèvement.
